# EUROPEAN PATENT APPLICATION

(11) **EP 4 477 740 A1**
(43) Date of publication of application: **18.12.2024**
(21) Application number: 23752925.0
(22) Date of filing: 08.02.2023
(51) Int. Cl.: C12N 1/21, C12N 15/54, C12P 19/18

(54) **METHOD FOR PRODUCING OLIGOSACCHARIDE HAVING LEWIS X SKELETON**

(30) Priority: 09.02.2022 JP 2022019024
(71) Applicant: KIRIN HOLDINGS KABUSHIKI KAISHA, Nakano-ku, Tokyo 164-0001 (JP)
(72) Inventor: SUGITA Tomotoshi, Tokyo 164-0001 (JP)
(74) Representative: Hoffmann Eitle
(86) International application number: PCT/JP2023/004262
(87) International publication number: WO 2023/153461

(57) **Abstract**

The present invention relates to a microorganism having an enhanced activity of a protein according to any one of the following [1] to [4] and improved productivity of an oligosaccharide having a Lewis X skeleton as compared with a parent strain: [1] a protein consisting of the amino acid sequence represented by SEQ ID NO: 2, [2] a mutant protein having an α1,3-fucosyl transferase activity and consisting of an amino acid sequence in which 1 to 20 amino acids are deleted, substituted, inserted, or added in the amino acid sequence represented by SEQ ID NO: 2, [3] a homologous protein having an α1,3-fucosyl transferase activity and consisting of an amino acid sequence having an identity of 90% or more with the amino acid sequence represented by SEQ ID NO: 2, and [4] a protein consisting of the amino acid sequence represented by SEQ ID NO: 24, 18, or 26.

## Description

### TECHNICAL FIELD

The present invention relates to a method for producing an oligosaccharide having a Lewis X skeleton.

### BACKGROUND ART

Oligosaccharides (HMO) contained in human breast milk have attracted attention as prebiotics materials, and effects such as development of cognitive functions of infants, infection protection, and improvement in intestinal environment have been disclosed (Non-Patent Literature 1).

Lacto-N-fucopentaose III (hereinafter, referred to as LNFPIII) and lacto-N-neodifucohexaose II (LNnDFHII) are known as one type of an oligosaccharide having a Lewis X skeleton. Among them, LNFPIII is a pentasaccharide HMO in which fucose is α1,3-linked to 3-position of N-acetylglucosamine in lacto-N-neotetraose (hereinafter, referred to as LNnT).

LNFPIII is abundant in human breast milk, after lacto-N-fucopentaose I (hereinafter, referred to as LNFPI) and lacto-N-fucopentaose II (hereinafter, referred to as LNFPII), which are also pentasaccharides and are known isomers of LNFPIII (Non-Patent Literature 2).

LNFPIII has been suggested to have an activation effect on macrophages and NK cells, and an effect on inducing secretion of cytokines such as IL-10 and TNFα (Non-Patent Literature 3), and functionality thereof is attracting attention.

An enzyme reaction method or a microbiological fermentation method using α1,3-fucose transferase is known as a method for producing an oligosaccharide having a Lewis X skeleton such as LNFPIII. As the α1,3-fucose transferase, for example, FucTIII derived from Helicobacter pylori (Non-Patent Literature 4) and Bf13FT derived from Bacteroides fragilis (Non-Patent Literature 5) are known.

Non-Patent Literature 6 discloses a method for producing LNFPIII by a fermentation method using Escherichia coli with overexpressing α1,3-fucose transferase, FutA, or FutB derived from Helicobacter pylori.

### CITATION LIST

### NON-PATENT LITERATURE

Non-Patent Literature 1: Int J Pediatrics (2019) 2390240: 1-8
Non-Patent Literature 2: Nutrients (2019) 11, 1282
Non-Patent Literature 3: Journal of Functional Foods 72 (2020) 104074
Non-Patent Literature 4: ACS Catal. (2019) 9, 10712-10720
Non-Patent Literature 5: ACS Catal. (2019) 9, 12, 11794-11800
Non-Patent Literature 6: Biotechnol. Prog. (2004) 20, 412-419
Non-Patent Literature 7: Metabolic Engineering 41 (2017) 23-38

### SUMMARY OF INVENTION

### TECHNICAL PROBLEM

As described above, the enzyme reaction method or the microbiological fermentation method using α1,3-fucose transferase is known as the method for producing an oligosaccharide having a Lewis X skeleton. However, since the known α1,3 -fucose transferase described in Non-Patent Literatures 4, 5, and 6 has low substrate specificity, for example, in production of LNFPIII, lacto-N-fucopentaose VI (hereinafter, referred to as LNFPVI) and lacto-N-neodifucohexaose II (hereinafter, referred to as LNnDFHII), in which fucose is linked to 3-position of glucose in LNnT on a reducing end side, may be produced as by-products.

Since the α1,3-fucose transferase derived from Helicobacter pylori described in Non-Patent Literature 6 can use not only LNnT but also lactose as a substrate, 3-fucosyl lactose (hereinafter, referred to as 3FL) is generated as a by-product (Non-Patent Literature 7). Therefore, it is not suitable for production of LNFPIII using lactose as an initial raw material.

In order to more efficiently produce an oligosaccharide having a Lewis X skeleton such as LNFPIII, there is a demand for searching for α1, 3-fucose transferase capable of selectively transferring fucose to an N-acetyl glucosamine site of a substrate such as LNnT.

Therefore, an object of the present invention is to provide a microorganism having excellent productivity of an oligosaccharide having a Lewis X skeleton, particularly a microorganism having excellent productivity of an oligosaccharide having an LNFPIII skeleton.

### SOLUTION TO PROBLEM

The present inventors have found that a microorganism having an enhanced activity of GDP-fucosyl transferase 1 (hereinafter, referred to as FucT1) of Parabacteroides goldsteinii JCM 13446, FucT-D of Gramella sp. BOM4, FucT-A of Parabacteroides sp. BX2, or FucT-E of Lachnospiraceae bacterium NLAE-zl-G231 has improved productivity of an oligosaccharide having a Lewis X skeleton as compared with a parent strain, and completed the present invention.

That is, the present invention is as follows.
1. A microorganism having an enhanced activity of a protein according to any one of the following [1] to [6] and improved productivity of an oligosaccharide having a Lewis X skeleton as compared with a parent strain:
   [1] a protein consisting of the amino acid sequence represented by SEQ ID NO: 2,
   [2] a mutant protein having an α1,3-fucosyl transferase activity and consisting of an amino acid sequence in which 1 to 20 amino acids are deleted, substituted, inserted, or added in the amino acid sequence represented by SEQ ID NO: 2,
   [3] a homologous protein having an α1,3-fucosyl transferase activity and consisting of an amino acid sequence having an identity of 90% or more with the amino acid sequence represented by SEQ ID NO: 2,
   [4] a protein consisting of the amino acid sequence represented by SEQ ID NO: 24,
   [5] a protein consisting of the amino acid sequence represented by SEQ ID NO: 18, and
   [6] a protein consisting of the amino acid sequence represented by SEQ ID NO: 26.
2. The microorganism according to the above 1, in which the oligosaccharide having a Lewis X skeleton is an oligosaccharide having a lacto-N-fucopentaose III (LNFPIII) skeleton.
3. The microorganism according to the above 2, in which the oligosaccharide having an LNFPIII skeleton is at least one of LNFPIII and lacto-N-neodifucohexaose II (LNnDFHII).
4. The microorganism according to the above 1, in which the oligosaccharide having a Lewis X skeleton is an oligosaccharide in which L-fucose is α1,3-linked to 3-position of at least one N-acetyl glucosamine contained in a paralacto-N-neohexaose (Para-LNnH) skeleton.
5. A method for producing an oligosaccharide, the method including: preparing the microorganism according to any one of the above 1 to 4; and producing an oligosaccharide in a culture using the microorganism.

### ADVANTAGEOUS EFFECTS OF INVENTION

Since the microorganism according to the present invention has an enhanced activity of a specific protein, fucose can be selectively transferred to an N-acetyl glucosamine site of a substrate, the production of by-products other than an oligosaccharide having a Lewis X skeleton can be reduced, and the productivity of the oligosaccharide having a Lewis X skeleton can be improved.

### BRIEF DESCRIPTION OF DRAWINGS

[FIG. 1] FIG. 1 shows a schematic diagram of a structure of Lewis X, LNFPIII, and paralacto-N-neohexaose (hereinafter, referred to as Para-LNnH).
[FIG. 2] FIG. 2 shows a schematic diagram of biosynthesis pathways of LNFPIII, LNnDFHII, and the like in a microorganism according to one embodiment of the present invention.
[FIG. 3] FIG. 3 shows an example of an oligosaccharide in which L-fucose is α1,3-linked to 3-position of at least one N-acetylglucosamine contained in a Para-LNnH skeleton.
[FIG. 4] FIG. 4 shows an alignment of the amino acid sequences represented by SEQ ID NOs: 18, 20, 22, 24, and 26 encoded by the PgsFucT1 homologue genes represented by SEQ ID NOs: 17, 19, 21, 23, and 25, respectively.
[FIG. 5] FIG. 5 shows a schematic diagram of biosynthesis pathways of 3FL and Lewis X.
[FIG. 6] FIG. 6 shows an alignment of the amino acid sequences encoding PgsFucT1, BfFucT1 and HpFutA, represented by SEQ ID NO: 2, SEQ ID NO: 12 and SEQ ID NO: 4, respectively.

### DESCRIPTION OF EMBODIMENTS

### 1. Microorganism Having Improved Productivity of Oligosaccharide having Lewis X Skeleton

Examples of a microorganism having improved productivity of an oligosaccharide having a Lewis X skeleton of the present invention include the following microorganism.

A microorganism having an enhanced activity of a protein according to any one of the following [1] to [6] and improved productivity of an oligosaccharide having a Lewis X skeleton as compared with a parent strain:
[1] a protein consisting of the amino acid sequence represented by SEQ ID NO: 2,
[2] a mutant protein having an α1,3-fucosyl transferase activity and consisting of an amino acid sequence in which 1 to 20 amino acids are deleted, substituted, inserted, or added in the amino acid sequence represented by SEQ ID NO: 2,
[3] a homologous protein having an α1,3-fucosyl transferase activity and consisting of an amino acid sequence having an identity of 90% or more with the amino acid sequence represented by SEQ ID NO: 2,
[4] a protein consisting of the amino acid sequence represented by SEQ ID NO: 24,
[5] a protein consisting of the amino acid sequence represented by SEQ ID NO: 18, and
[6] a protein consisting of the amino acid sequence represented by SEQ ID NO: 26.

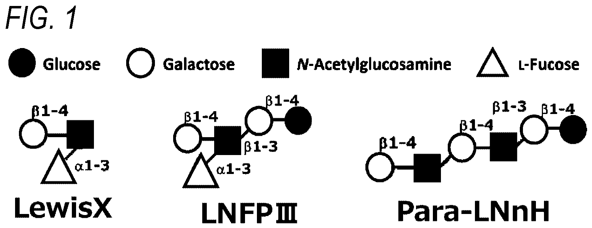

In the present description, the oligosaccharide having a Lewis X skeleton may be any oligosaccharide as long as it has a Lewis X skeleton consisting of Gal-β1,4(Fuc-α1,3)GlcNAc shown in FIG. 1. The oligosaccharide having a Lewis X skeleton is preferably an oligosaccharide having an LNFPIII skeleton or an oligosaccharide in which L-fucose is α1,3-linked to 3-position of at least one N-acetylglucosamine contained in a Para-LNnH skeleton.

In the present description, the oligosaccharide having an LNFPIII skeleton may be any oligosaccharide as long as it has an LNFPIII skeleton, and LNFPIII or LNnDFHII is preferred, and LNFPIII is more preferred.

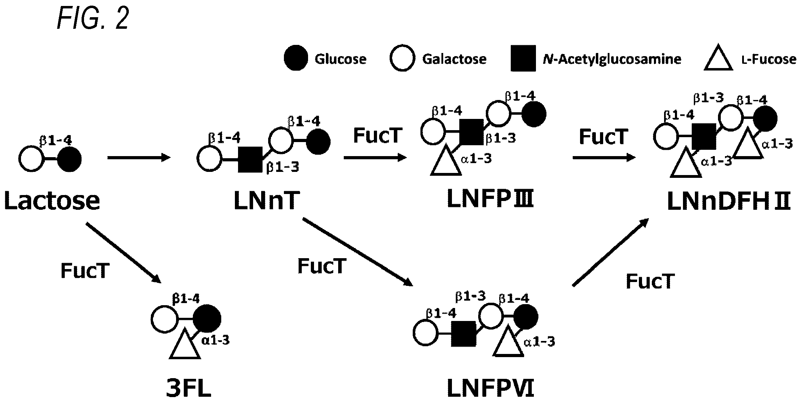

FIG. 1 shows a schematic diagram of a structure of LNFPIII. FIG. 2 shows a schematic diagram of biosynthesis pathways of LNFPIII, LNnDFHII, and the like in a microorganism according to one embodiment of the present invention.

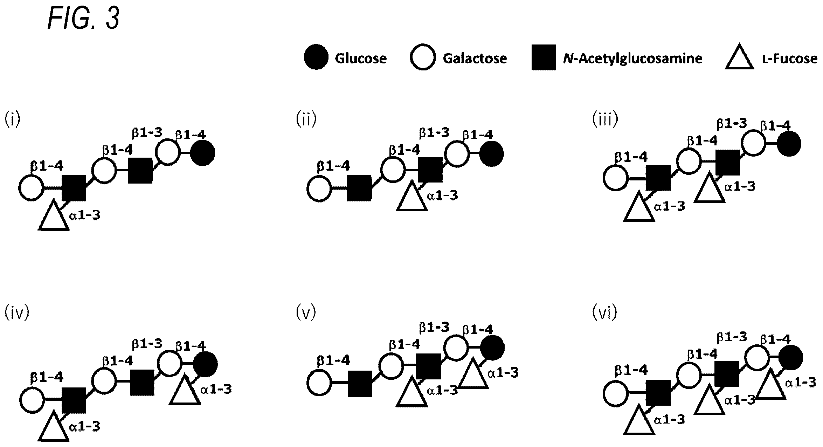

In the present description, examples of the oligosaccharide in which L-fucose is α1,3-linked to 3-position of at least one N-acetylglucosamine contained in a Para-LNnH skeleton include an oligosaccharide having a structure shown in FIG. 3. FIG. 1 shows a schematic diagram of a structure of Para-LNnH.

In the present description, the mutant protein refers to a protein obtained by artificially deleting or substituting an amino acid residue of an original protein or artificially inserting or adding an amino acid residue in the protein.

The expression "an amino acid is deleted, substituted, inserted, or added" in the mutant protein of the above [2] may mean that 1 to 20 amino acids are deleted, substituted, inserted, or added at any position in the same sequence. The number of amino acids to be deleted, substituted, inserted, or added is 1 to 20, preferably 1 to 10, more preferably 1 to 8, and most preferably 1 to 5.

An amino acid to be deleted, substituted, inserted, or added may be natural or non-natural. Examples of the natural amino acid include L-alanine, L-asparagine, L-aspartic acid, L-glutamine, L-glutamic acid, glycine, L-histidine, L-isoleucine, L-leucine, L-lysine, L-arginine, L-methionine, L-phenylalanine, L-proline, L-serine, L-threonine, L-tryptophan, L-tyrosine, L-valine, and L-cysteine.

Examples of mutually substitutable amino acids are shown below. Amino acids contained in the same group can be mutually substituted.
Group A: leucine, isoleucine, norleucine, valine, norvaline, alanine, 2-aminobutanoic acid, methionine, O-methylserine, t-butylglycine, t-butylalanine, and cyclohexylalanine
Group B: aspartic acid, glutamic acid, isoaspartic acid, isoglutamic acid, 2-aminoadipic acid, and 2-aminosuberic acid
Group C: asparagine and glutamine
Group D: lysine, arginine, ornithine, 2,4-diaminobutanoic acid, and 2,3-diaminopropionic acid
Group E: proline, 3-hydroxyproline, and 4-hydroxyproline
Group F: serine, threonine, and homoserine
Group G: phenylalanine and tyrosine

In the mutant protein of the above [2], examples of the amino acid residue to be substituted include an asparagine residue at position 17.

In the present description, the homologous protein is a protein for which a gene encoding the protein is thought to have the same evolutionary origin as a gene encoding an original protein due to similarity in structure and function with the original protein, and is a protein possessed by organisms exiting in nature.

Examples of the homologous protein include those with an amino acid sequence having an identity of 90% or more, preferably 93% or more, more preferably 95% or more, and particularly preferably 97% or more with the amino acid sequence of a target protein.

The identity of an amino acid sequence and a nucleotide sequence can be determined by using an algorithm BLAST [Pro. Natl. Acad. Sci. USA, 90, 5873 (1993)] or FASTA [Methods Enzymol., 183, 63 (1990)] developed by Karlin and Altschul. Programs called BLASTN and BLASTX have been developed based on the algorithm BLAST [J. Mol. Biol., 215, 403 (1990)]. When the nucleotide sequence is analyzed by BLASTN based on BLAST, the parameters are, for example, Score = 100 and wordlength = 12. When the amino acid sequence is analyzed by BLASTX based on BLAST, the parameters are, for example, score = 50 and wordlength = 3. When BLAST and Gapped BLAST programs are used, default parameters for each program are used. A specific method of the analysis methods is well known.

In the present description, the α1,3-fucosyl transferase activity refers to an activity of transferring fucose from GDP-fucose, which is a donor substrate, to an N-acetyl glucosamine 3-hydroxyl group of a carbohydrate, which is a receptor substrate, (hereinafter, referred to as a "receptor carbohydrate") by an α1,3-bond to produce a fucose-containing carbohydrate.

Examples of the receptor carbohydrate include a compound having an N-acetyllactosamine (hereinafter, referred to as LacNAc) skeleton, and examples thereof include LNnT, LNFPVI, para-LNnH, a combination thereof, and a sugar chain containing these as a partial structure. Among them, LNnT is preferred.

In the present description, the term "receptor substrate" refers to substances on which α1,3-fucosyl transferase can act to produce an oligosaccharide having a Lewis X skeleton, or a combination thereof.

In the present invention, a parent strain refers to an original strain to be subjected to genetic modification, transformation, and the like. In particular, a parent strain of a microorganism having improved productivity of an oligosaccharide having a Lewis X skeleton of the present invention refers to a strain before genetic modification and transformation, and the like to enhance the activity of the protein according to any one of [1] to [6].

In the present description, the parent strain is preferably a prokaryote or a yeast strain, more preferably a prokaryote belonging to the genus Escherichia, the genus Serratia, the genus Bacillus, the genus Brevibacterium, the genus Corynebacterium, the genus Microbacterium, the genus Pseudomonas, or the like, or a yeast strain belonging to the genus Saccharomyces, the genus Schizosaccharomyces, the genus Kluyveromyces, the genus Trichosporon, the genus Siwaniomyces, the genus Pichia, the genus Candida, or the like, and most preferably a prokaryote such as Escherichia coli MG1655, Escherichia coli XL 1 -Blue, Escherichia coli XL2-Blue, Escherichia coli DH1, Escherichia coli MC1000, Escherichia coli KY3276, Escherichia coli W1485, Escherichia coli JM109, Escherichia coli HB101, Escherichia coli No. 49, Escherichia coli W3110, Escherichia coli NY49, Escherichia coli BL21 codon plus (manufactured by Stratagene Corporation), Escherichia coli W3110S3GK (NBRC114657), Serratia ficaria, Serratia fonticola, Serratia liquefaciens, Serratia marcescens, Bacillus subtilis, Bacillus amyloliquefaciens, Brevibacterium immariophilum ATCC14068, Brevibacterium saccharolyticum ATCC14066, Cory nebacterium ammoniagenes, Corynebacterium glutamicum ATCC13032, Corynebacterium glutamicum ATCC14067, Corynebacterium glutamicum ATCC13869, Corynebacterium acetoacidophilum ATCC13870, Microbacterium ammoniaphilum ATCC15354, or Pseudomonas sp. D-0110, or a yeast strain such as Saccharomyces cerevisiae, Schizosaccharomyces pombe, Kluyveromyces lactis, Trichosporon pullulans, Schwanniomyces alluvius, Pichia pastoris, or Candida utilis.

The parent strain may be a wild strain as long as it is a microorganism that produces GDP-fucose and/or a receptor carbohydrate. When a wild strain does not have an ability to produce GDP-fucose and/or a receptor carbohydrate, the wild strain may be a breeding strain artificially endowed with an ability to supply GDP-fucose and/or a receptor carbohydrate.

### 1) A microorganism used as a parent strain and having an artificially endowed or enhanced ability to supply GDP-fucose, which is a reaction substrate for α1,3-fucosyl transferase

The parent strain is preferably a microorganism having an artificially endowed or enhanced ability to supply GDP-fucose, which is a reaction substrate for α1,3-fucosyl transferase. Specific examples of a method for endowing or enhancing the ability to supply GDP-fucose to a microorganism used as a parent strain include a known method such as a method using various genetic manipulations (Metabolic Engineering (2017) 41: 23-38).

Examples of the ability to produce GDP-fucose include an ability to produce GDP-fucose from a saccharide. Examples of the method for artificially endowing or enhancing the ability to supply GDP-fucose from a saccharide to a microorganism used as a parent strain include the following methods (1a) to (1d). These methods may be used alone or in combination.
(1a) A method of alleviating or releasing at least one mechanism for controlling a biosynthesis pathway for producing GDP-fucose from a saccharide.
(1b) A method of enhancing expression of at least one enzyme associated with the biosynthesis pathway for producing GDP-fucose from a saccharide.
(1c) A method of increasing the number of copies of at least one gene encoding an enzyme associated with the biosynthesis pathway for producing GDP-fucose from a saccharide.
(1d) A method of weakening or blocking at least one metabolic pathway branching off from the biosynthesis pathway for producing GDP-fucose from a saccharide to a metabolic product other than a target substance.

Specific examples of the mechanism for controlling a biosynthesis pathway for producing GDP-fucose from a saccharide include known mechanisms such as a control mechanism based on a transcription control factor (e.g., RcsA) associated with control of the biosynthesis pathway. RcsA is a regulatory factor for uppreforming the entire colanic acid biosynthesis pathway using GDP-fucose as an intermediate. As will be described later, by strengthening rcsA in a state where a pathway downstream of GDP-fucose in the colanic acid biosynthesis pathway is blocked, a large amount of GDP-fucose can be accumulated.

Specific examples of the enzyme associated with the biosynthesis pathway for producing GDP-fucose from a saccharide include known enzymes such as a mannose-6-phosphate isomerase, a phosphomannomutase, a mannose-1-phosphate guanylyltransferase, a GDP mannose-4,6-dehydratase, and a GDP-L-fucose synthase.

Specific examples of the metabolic pathway branching off from a biosynthesis pathway for producing GDP-fucose from a saccharide to a metabolic product other than a target substance include a known metabolic pathway such as a metabolic pathway from GDP-fucose to colanic acid. In particular, the supply of GDP-fucose can be enhanced by blocking WcaJ, WzxC, WcaK, WcaL, or WcaM, which is a pathway downstream of GDP-fucose in the colanic acid biosynthesis pathway.

### 2) A microorganism used as a parent strain and having an artificially endowed or enhanced ability to supply a receptor carbohydrate, which is a reaction substrate for α1,3-fucosyl transferase

Examples of a method for artificially endowing the ability to supply a receptor carbohydrate to a microorganism used as a parent strain include the following methods (2a) to (2h), and these methods can be used alone or in combination.
(2a) A method of alleviating or releasing at least one mechanism for controlling a biosynthesis pathway for producing a receptor carbohydrate from a saccharide.
(2b) A method of enhancing expression of at least one enzyme associated with the biosynthesis pathway for producing a receptor carbohydrate from a saccharide.
(2c) A method of increasing the number of copies of at least one enzyme gene associated with the biosynthesis pathway for producing a receptor carbohydrate from a saccharide.
(2d) A method of alleviating or releasing at least one mechanism for decomposing a receptor carbohydrate or a saccharide that is a substrate thereof.
(2e) A method of enhancing an expression of at least one enzyme associated with intracellular uptake of a receptor carbohydrate or a saccharide that is a substrate thereof.
(2f) A method of increasing the number of copies of at least one gene encoding an enzyme associated with intracellular uptake of a receptor carbohydrate or a saccharide that is a substrate thereof.
(2g) A method of weakening or blocking at least one metabolic pathway branching off from the biosynthesis pathway for producing a receptor carbohydrate from a saccharide to a metabolic product other than a target substance.
(2h) A method of selecting a cell strain having resistance to a receptor carbohydrate higher than that of a wild-type strain.

Specific examples of the enzyme associated with the biosynthesis pathway for producing a receptor carbohydrate from a saccharide include known enzymes such as an enzyme associated with a biosynthesis pathway for producing LNnT from glucose and lactose and having a β1,4-galactosyltransferase (hereinafter, referred to as "galT") activity and an enzyme having a β1,3-N-acetylglucosamine transferase (hereinafter, referred to as "LgtA") activity.

Specific examples of the mechanism for decomposing a receptor carbohydrate or a saccharide as a substrate thereof include known enzymes such as β-galactosidase that catalyze hydrolysis of lactose as a substrate for LNnT to produce glucose and galactose. Specific examples thereof include β-galactosidase (hereinafter, referred to as lacZ) that hydrolyzes lactose which is a substrate for LNTII, and a decrease in supply of lactose can be prevented by losing the activity of lacZ.

Specific examples of the enzyme associated with intracellular uptake of a receptor carbohydrate or a saccharide as a substrate thereof include known enzymes such as a lactose permease associated with intracellular uptake of lactose which is a substrate for LNnT.

Specifically, for example, in order to supply LNnT, the microorganism having an endowed or enhanced ability to supply a receptor carbohydrate preferably has at least one activity selected from a lactose permease (hereinafter, referred to as lacY) activity, a β1,4-galactosyltransferase (hereinafter, referred to as galT) activity, a β1,3-N-acetylglucosamine transferase (hereinafter, referred to as LgtA) activity, a glutamine fructose-6-phosphate transaminase (hereinafter, referred to as glmS) activity, a phosphoglucosamine mutase (hereinafter, referred to as glmM) activity, a N-acetylglucosamine-1-phosphate uridyltransferase/glucosamine-1-phosphate acetyltransferase (hereinafter, referred to as glmU) activity, a phosphoglucomutase (hereinafter, referred to as Pgm) activity, an UTP glucose-1-phosphate uridyltransferase (hereinafter, referred to as galU) activity, an UDP glucose-4-epimerase (hereinafter, referred to as galE) activity, an UTP glucose-1-phosphate uridyltransferase (hereinafter, referred to as galF) activity, a glucose-6-phosphate isomerase (hereinafter, referred to as Pgi) activity, and more preferably has enhanced activities thereof.

Among them, the microorganism preferably has lacY, galT, and lgtA activities, and more preferable has enhanced activities thereof.

lacY is a membrane protein that takes up lactose which is a substrate for a receptor carbohydrate into cells. galT is an enzyme associated with generation of LNnT from lacto-N-triose II (LNTII). LNnT is a precursor of LNFPIII. LgtA is an enzyme associated with production of LNTII from lactose and uridine diphosphate-N-acetylglucosamine (hereinafter, referred to as UDP-GlcNAc). LNTII is a precursor of LNnT.

glmS, glmM, and glmU are enzymes associated with a biosynthesis pathway for producing LNTII. Pgm, galU, galE, and galF are enzymes associated with a pathway for producing uridine diphosphate galactose (hereinafter, referred to as UDF-Gal). Pgi is an enzyme associated with a pathway for producing LNTII.

The fact that the microorganism is a microorganism capable of producing a receptor carbohydrate and/or GDP-fucose can be confirmed by culturing the microorganism in a culture medium and detecting a receptor carbohydrate and/or GDP-fucose accumulated in a culture by using a general method such as a carbohydrate analyzer or a high performance liquid chromatograph mass spectrometer to be described later.

The microorganism used as a parent strain of the present invention is preferably a microorganism having an artificially endowed or enhanced ability to supply GDP-fucose and/or a receptor carbohydrate, which is a reaction substrate for α1,3-fucosyl transferase. Therefore, in one embodiment of the present invention, the parent strain is preferably a genetically modified microorganism comprising at least one nucleotide sequence selected from the nucleotide sequence encoding rcsA (Accession Number: BAA15776.1), a nucleotide sequence encoding mannose-6-phosphate isomerase (Accession Number: BAA15361.1), a nucleotide sequence encoding phosphomannomutase (Accession Number: BAA15901.1), a nucleotide sequence encoding mannose-1-phosphate guanylyltransferase (Accession Number: BAA15905.1), a nucleotide sequence encoding GDP mannose-4,6-dehydratase (Accession Number: BAA15909.1), a nucleotide sequence encoding GDP-L-fucose synthase (Accession Number: BAA15908.1), a nucleotide sequence encoding lacY (Accession Number: BAE76125.1), a nucleotide sequence encoding galT (SEQ ID NO: 29), a nucleotide sequence encoding LgtA (SEQ ID NO: 31), a nucleotide sequence encoding glmS (Accession Number: BAE77559.1), a nucleotide sequence encoding glmM (Accession Number: BAE77220.1), a nucleotide sequence encoding glmU (Accession Number: BAE77558.1), a nucleotide sequence encoding Pgm (Accession Number: BAA35337.1), a nucleotide sequence encoding galU (Accession Number BAA36104.1), a nucleotide sequence encoding galE (Accession Number: BAA35421.1), a nucleotide sequence encoding galF (Accession Number: BAA15896.1), and a nucleotide sequence encoding Pgi (Accession Number: BAE78027.1).

In particular, the parent strain is more preferably a genetically modified microorganism comprising the nucleotide sequence encoding lacY, a nucleotide sequence encoding rcsA, a nucleotide sequence encoding galT, and a nucleotide sequence encoding lgtA. In one embodiment of the present invention, the genetically modified microorganism preferably has an enhanced ability to produce GDP-fucose and/or a receptor substrate as compared with a parent strain that is not genetically modified.

A known method may be used as a method for producing a microorganism having at least one activity selected from a lacY activity, a rcsA activity, a galT activity, a LgtA activity, a glmS activity, a glmM activity, a glmU activity, a Pgm activity, a galU activity, a galE activity, a galF activity, and a Pgi activity, or having an enhanced activity thereof. Specific examples thereof include methods using various genetic manipulations (Syst Microbiol Biomanufact, 2021, 1, 291).

Further, as described above, the parent strain preferably has a reduced or deleted lacZ activity and/or colanic acid synthesis activity.

Accordingly, in one embodiment of the present invention, the parent strain is preferably a genetically modified microorganism having a reduced or deleted lacZ activity and/or colanic acid synthesis activity, and more preferably a genetically modified microorganism not containing the nucleotide sequence encoding lacZ and/or the nucleotide sequence encoding a wcaJ, wzxC, wcaK, wcaL, or wcaM gene which is a nucleotide sequence encoding a protein associated with colanic acid synthesis.

In one embodiment of the present invention, the genetically modified microorganism preferably has an enhanced ability to produce a receptor carbohydrate and/or GDP-fucose as compared with a parent strain that is not genetically modified.

A known method may be used as a method for producing Escherichia coli having a reduced or deleted β-galactosidase activity and/or colanic acid synthesis activity. Specific examples include methods using various genetic manipulations (Metabolic Engineering, 2017, 41:23-38).

Examples of the microorganism having an enhanced activity of the protein according to any one of the above [1] to [6] as compared with the microorganism of the parent strain include a microorganism having an increased copy number of the gene as compared with the parent strain, which is obtained by transforming the microorganism of the parent strain with a recombinant DNA containing a DNA encoding the protein.

Examples of the microorganism having an increased copy number of the gene as compared with the parent strain, which is obtained by transforming the microorganism of the parent strain with a recombinant DNA containing a DNA encoding the protein according to any one of the above [1] to [6] include a microorganism having an increased copy number of the gene on a chromosomal DNA by transforming the microorganism of the parent strain with the recombinant DNA containing the DNA encoding the protein according to any one of the above [1] to [6], and a microorganism carrying the above gene outside of a chromosomal DNA as a plasmid DNA.

The DNA encoding the protein according to any one of the above [1] to [6] may be any DNA as long as it encodes a protein having an activity of the protein according to any one of [1] to [6]. Specific examples thereof include one DNA selected from the group consisting of the following [7] to [10].
[7] A DNA encoding the protein according to any one of the above [1] to [6].
[8] A DNA consisting of the nucleotide sequence represented by SEQ ID NO: 1, 23, 17, or 25.
[9] A DNA hybridizing with a DNA consisting of a nucleotide sequence complementary to the nucleotide sequence represented by SEQ ID NO: 1, 23, 17, or 25 under stringent conditions and encoding a homologous protein having an α1,3-fucosyl transferase activity.
[10] A DNA consisting of a nucleotide sequence having an identity of 95% or more, preferably 97% or more, more preferably 98% or more, and most preferably 99% or more with the nucleotide sequence represented by SEQ ID NO: 1, 23, 17, or 25 and encoding a homologous protein having an α1,3-fucosyl transferase activity.

In the above [9], the term "hybridizing" means that a DNA hybridizes to a DNA having a specific nucleotide sequence or a part of the DNA. Accordingly, the DNA having a specific nucleotide sequence or a part thereof is a DNA that can be used as a probe for Northern or Southern blot analysis, or a DNA that can be used as an oligonucleotide primer for PCR analysis.

Examples of the DNA used as a probe include DNAs having at least 100 bases or more, preferably 200 bases or more, and more preferably 500 bases or more. Examples of the DNA used as a primer include DNAs having at least 10 bases or more, and preferably 15 bases or more.

A method for a hybridization experiment of a DNA is well known, and for example, those skilled in the art can determine hybridization conditions according to the present description. The hybridization conditions can be determined according to those described in Molecular Cloning, 4th Edition (2012), Methods for General and Molecular Bacteriology, ASM Press (1994), and Immunology methods manual, Academic press (1996) as well as many other standard textbooks.

The DNA hybridizing under stringent conditions can also be obtained by following an explanatory manual attached to a commercially available hybridization kit. Examples of the commercially available hybridization kit include a random primed DNA labeling kit (manufactured by Roche Diagnostics K.K.) in which a probe is prepared by a random prime method and hybridization is performed under stringent conditions.

Examples of the above stringent conditions include conditions where a filter on which a DNA is immobilized and a probe DNA are incubated overnight at 42°C in a solution containing 50% formamide, 5×SSC (750 mmol/L sodium chloride, 75 mmol/L sodium citrate), 50 mmol/L sodium phosphate (pH 7.6), a 5× Denhardt's solution, 10% dextran sulfate, and a 20 µg/l of denatured salmon sperm DNA, and then the filter is washed in a 0.2×SSC solution at, for example, about 65°C.

Examples of the DNA capable of hybridizing under the above stringent conditions include DNAs having an identity of at least 95% or more, preferably 97% or more, more preferably 98% or more, and most preferably 99% or more with the DNA consisting of the nucleotide sequence represented by SEQ ID NO: 1, 23, 17, or 25 when calculated based on the parameters or the like using BLAST, FASTA, or the like.

The DNA encoding the protein of the above [1], [4], [5], or [6] and the DNA of the above [8] can be obtained by, for example, Southern hybridization for a chromosomal DNA library of a microorganism, preferably a microorganism belonging to the genus Escherichia coli, and more preferably an Escherichia coli W3110 strain using a probe DNA that can be designed based on the nucleotide sequence represented by SEQ ID NO: 1 (FucT1 gene), a gene represented by SEQ ID NO: 23 (FucT-D gene), a gene represented by SEQ ID NO: 17 (FucT-A gene), or a nucleotide sequence represented by SEQ ID NO: 25 (FucT-E gene), or PCR [PCR protocols, Academic Press (1990)] using, as a template, a chromosomal DNA of a microorganism using a primer DNA that can be designed based on the nucleotide sequence.

The Escherichia coli W3110 strain can be available from the National Institute of Technology and Evaluation (NITE) Biological Resource Center.

The DNA encoding the mutant protein of the above [2] can be obtained by, for example, performing error-prone PCR, or the like, using, as a template, a DNA consisting of the nucleotide sequence represented by SEQ ID NO: 1, 23, 17, or 25.

Alternatively, the DNA in the above [2] can also be obtained by PCR [Gene, 77, 51 (1989)] using a set of PCR primers each having a nucleotide sequence designed to insert a target mutation (deletion, substitution, insertion, or addition) at the 5' end.

The DNA can also be obtained by following an explanatory manual attached to a commercially available partially directed mutagenesis kit. Examples of the commercially available partially directed mutagenesis kit include a PrimeSTAR (registered trademark) Mutagenesis Basal Kit (manufactured by Takara Bio Inc.) capable of introducing a mutation (deletion, substitution, insertion, or addition) at a position to which a desired mutation is to be introduced.

That is, first, a pair of mutagenesis primers having a 15-base overlap on the 5' side is designed using a plasmid comprising a nucleotide sequence designed to introduce a desired mutation (deletion, substitution, insertion, or addition) as a template. At this time, the overlap portion includes a desired mutation. Next, PCR is performed using the mutagenesis primers and using a plasmid comprising a nucleotide sequence into which a desired mutation is introduced as a template. When the amplified fragment thus obtained is transformed into Escherichia coli, a plasmid comprising a nucleotide sequence into which a desired mutation is introduced is obtained.

The DNA encoding the homologous protein of the above [3] and the DNA of the above [9] and [10] can be obtained, for example, by a method same as the above method for obtaining the DNA by, for example, searching various gene sequence databases for a nucleotide sequence having an identity of 95% or more, preferably 97% or more, further preferably 98% or more, and most preferably 99% or more with the nucleotide sequence represented by SEQ ID NO: 1, 23, 17, or 25 searching various protein sequence databases for an amino acid sequence having an identity of 95% or more, preferably 97% or more, further preferably 98% or more, and most preferably 99% or more with the amino acid sequence represented by SEQ ID NO: 2, 24, 18, or 26, and using a probe DNA or a primer DNA that can be designed based on the nucleotide sequence or the amino acid sequence obtained by the search, and a microorganism having the DNA.

A nucleotide sequence of the obtained DNA according to any one of [7] to [10] can be determined by incorporating the DNA as is or the DNA cleaved with an appropriate restriction enzyme or the like into a vector by a common method, introducing the obtained recombinant DNA into a host cell, and then analyzing the DNA using a nucleotide sequence analysis method generally used, such as a dideoxy method [Proc. Natl. Acad. Sci., USA, 74, 5463 (1977)] or a nucleotide sequence analyzer such as an Applied Biosystems 3500 Genetic Analyzer and an Applied Biosystems 3730 DNA Analyzer (both manufactured by Thermo Fisher Scientific K.K.).

The host cell that can be used for determining the nucleotide sequence of the DNA may be any cell as long as the vector can be introduced and proliferated, and examples thereof include Escherichia coli DH5α, Escherichia coli HST08 Premium, Escherichia coli HST02, Escherichia coli HST04 dam⁻/dcm⁻, Escherichia coli JM109, Escherichia coli HB101, Escherichia coli CJ236, Escherichia coli BMH71-18 mutS, Escherichia coli MV1184, and Escherichia coli TH2 (all manufactured by Takara Bio Inc.), Escherichia coli XL1-Blue and Escherichia coli XL2-Blue (both manufactured by Agilent Technologies, Inc.), Escherichia coli DH1, Escherichia coli MC1000, Escherichia coli W1485, Escherichia coli W3110, Escherichia coli MP347, and Escherichia coli NM522.

Examples of the above vector include pBluescriptII KS(+) and pPCR-Script Amp SK(+) (both manufactured by Agilent Technologies, Inc.), pT7Blue (manufactured by Merck Millipore Inc.), pCRII (manufactured by Thermo Fisher Scientific K.K.), pCR-TRAP (manufactured by Gene Hunter), and pDIRECT (Nucleic Acids Res., 18, 6069, 1990).

As a method for introducing the recombinant DNA, any method for introducing a DNA into a host cell can be used, and examples thereof include a method using calcium ions [Proc. Natl. Acad. Sci., USA, 69, 2110 (1972)], a protoplast method (JPS63-248394A), and an electroporation method [Nucleic Acids Res., 16, 6127 (1988)].

When the obtained DNA is partial length as a result of determining the nucleotide sequence, the full-length DNA can be obtained by a Southern hybridization method or the like for a chromosomal DNA library using the partial-length DNA as a probe.

Further, a target DNA can also be prepared by chemical synthesis using an NTS M series DNA synthesizer or the like manufactured by Nihon Techno Service Co., Ltd. based on the determined DNA nucleotide sequence.

A recombinant DNA containing the DNA encoding the protein according to any one of the above [1] to [6] refers to a recombinant DNA obtained by incorporating the DNA into an expression vector which is autonomously replicable in a parent strain or can be incorporated into a chromosome and contains a promoter at a position where the DNA can be transferred.

When the recombinant DNA is a recombinant DNA capable of being incorporated into a chromosome, the recombinant DNA may not contain a promoter

The microorganism having an increased copy number of the gene as compared with the parent strain, which is obtained by transforming the microorganism of the parent strain with the recombinant DNA containing the DNA encoding the protein according to any one of the above [1] to [6] can be obtained by the following method.

Based on the DNA encoding the protein according to any one of the above [1] to [6] and obtained by the above method, a DNA fragment of an appropriate length containing a portion encoding the protein is prepared as necessary. A transformant having an improved production rate can be obtained by substituting a base such that a nucleotide sequence of the portion encoding the protein becomes an optimal codon for expression in a host cell.

A recombinant DNA is prepared by inserting the DNA fragment downstream of a promoter of an appropriate expression vector. By transforming the parent strain with the recombinant DNA, a microorganism having an increased copy number of a gene encoding the protein as compared with the parent strain can be obtained.

When a prokaryote such as bacteria is used as parent strain, the recombinant DNA is preferably a recombinant DNA composed of a promoter, a ribosomal binding sequence, the DNA according to any one of the above [7] to [10], and a transcription termination sequence. A gene for regulating the promoter may be contained.

It is preferable to use a plasmid in which a distance between a Shine-Dalgarno sequence, which is a ribosome binding sequence, and an initiation codon is adjusted to an appropriate distance (for example, 6 to 18 bases). In the recombinant DNA, a transcription termination sequence is not necessarily required for expression of the DNA, but it is preferable to place the transcription termination sequence immediately after a structural gene.

An expression level of the protein having an α1,3-fucosyl transferase activity can be improved by substituting a base such that the nucleotide sequence of the portion encoding the protein having an α1,3-fucosyl transferase activity becomes an optimal codon for host expression. Examples of the protein having an α1,3-fucosyl transferase activity include the protein according to any one of the above [1] to [6]. Information on the frequency of codon use in the parent strain used in the present invention can be obtained through a public database.

The expression vector is not particularly limited as long as it is an appropriate nucleic acid molecule for introducing the target DNA into a host and causing the target DNA to be amplified and expressed. Not only plasmids, but also, for example, artificial chromosomes, vectors using transposons, and cosmids may be used.

When a microorganism belonging to the genus Escherichia is used as the parent strain, examples of the expression vector include pColdI, pSTV28, pSTV29, pUC118 (all manufactured by Takara Bio Inc.), pMW118 and pMW119 (all manufactured by Nippon Gene Co., ltd.), pET21a, pCOLADuet-1, pCDFDuet-1, pCDF-1b, pRSF-1b (all manufactured by Merck Millipore Inc.), pMAL-c5x (manufactured by New England Biolabs), pGEX-4T-1, pTrc99A (both manufactured by GE Healthcare Bioscience), pTrcHis, pSE280 (both manufactured by Thermo Fisher Scientific K.K.), pGEMEX-1 (manufactured by Promega Corporation), pQE-30, pQE80L (both manufactured by Qiagen), pET-3, pBluescriptII SK(+), pBluescriptII KS(-) (all manufactured by Agilent Technologies, Inc.), pUAKQE31 (Appl. Environ. Microbiol. 2007, 73: 6378-6385), pKYP10 (JPS58-110600A), pKYP200 [Agric. Biol. Chem., 48, 669 (1984)], pLSA1 [Agric. Biol. Chem., 53, 277 (1989)], pGEL1 [Proc. Natl. Acad. Sci., USA, 82, 4306 (1985)], pBluescript II SK(+), pBluescript II KS(-) (manufactured by Stratagene Corporation), pTrS30 [prepared from Escherichia coli JM109/pTrS30 (FERM BP-5407)], pTrS32 [prepared from Escherichia coli JM109/pTrS32 (FERM BP-5408)], pTK31 [APPLIED AND ENVIRONMENTAL MICROBIOLOGY, 2007, Vol.73, No.20, p 6378-6385], pPAC31 (WO1998/12343), pUC19 [Gene, 33, 103 (1985)], pPA1 (JPS63-233798A), and pKD46 [Proc. Natl. Acad. Sci., USA, 97, 6640-6645 (2000)].

The promoter in the case of using the above expression vector may be any promoter as long as it functions in cells of microorganisms belonging to the genus Escherichia, and for example, a promoter of a gene associated with amino acid biosynthesis, such as a trp promoter or an ilv promoter, and a promoter derived from, for example, an Escherichia coli or a phage, such as a uspA promoter, a lac promoter, a PL promoter, a PR promoter, or a PSE promoter can be used. Examples thereof include a promoter which is artificially modified in design, such as a promoter with two trp promoters in series, a tac promoter, a trc promoter, a lacT7 promoter, or a letI promoter.

When a microorganism belonging to the genus Corynebacterium is used as the parent strain, examples of the expression vector include pCG1 (JPS57-134500A), pCG2 (JPS58-35197A), pCG4 (JPS57-183799A), pCG11 (JPS57-134500A), pCG116, pCE54, and pCB101 (all JPS58-105999A), pCE51, pCE52, and pCE53 [all Molecular and General Genetics, 196, 175, (1984)].

When the above expression vector is used, the promoter may be any promoter as long as it functions in cells of microorganisms belonging to the genus Corynebacterium, and for example, a P54-6 promoter [Appl. Microbiol. Biotechnol., 53, 674-679 (2000)] can be used.

When a yeast strain is used as the parent strain, examples of the expression vector include YEp13 (ATCC37115), YEp24 (ATCC37051), YCp50 (ATCC37419), pHS19, and pHS15.

The promoter in the case of using the above expression vector may be any promoter as long as it functions in cells of the yeast strain, and examples thereof include a PHOS promoter, a PGK promoter, a GAP promoter, an ADH promoter, a gall promoter, a gal10 promoter, a heat shock polypeptide promoter, an MFα1 promoter, and a CUP 1 promoter.

By inserting the DNA fragment according to any one of the above [7] to [10] downstream of a promoter of an appropriate expression vector, a recombinant DNA to be used in the production method of the present invention can be prepared.

Examples of a method for introducing a recombinant DNA into a parent strain as an autonomously replicable plasmid include a method using calcium ions [Proc. Natl. Acad. Sci., USA, 69, 2110 (1972)], a protoplast method (JPS63-248394A), and an electroporation method [Nucleic Acids Res., 16, 6127 (1988)].

Examples of the method for incorporating a recombinant DNA into a chromosome of a host cell include a homologous recombination method. Examples of the homologous recombination method include a method using a plasmid for homologous recombination that can be prepared by linking with a plasmid DNA having a drug resistance gene that cannot autonomously replicate in a host cell to be introduced. Examples of the method using homologous recombination frequently used in Escherichia coli include a method of introducing a recombinant DNA using a homologous recombination system of a lambda phage [Proc. Natl. Acad. Sci. USA, 97, 6640-6645 (2000)].

Further, by using a selection method based on the fact that Escherichia coli becomes sucrose-sensitive due to a Bacillus subtilis revansucrase incorporated on the chromosome together with a recombinant DNA, or a selection method based on the fact that Escherichia coli becomes streptomycin-sensitive by incorporating a wild-type rpsL gene into Escherichia coli comprising a mutant rpsL gene for streptomycin resistance [Mol. Microbiol., 55, 137 (2005), Biosci. Biotechnol. Biochem., 71, 2905 (2007)], Escherichia coli with a target region on a chromosomal DNA of the host cell substituted with the recombinant DNA can be obtained.

The fact that the recombinant DNA is introduced into the parent strain as an autonomously replicable plasmid or incorporated into a chromosome of the parent strain can be confirmed by, for example, a method in which a gene originally contained in a chromosomal DNA of a microorganism cannot be amplified, but the gene introduced through transformation can be amplified by PCR using a primer set to confirm an amplification product. The fact that the transcription amount of the DNA or the production amount of the protein encoded by the DNA is increased can be confirmed by a method in which the transcription amount of the gene of the microorganism is measured by Northern blotting, or the production amount of the protein of the microorganism is measured by Western blotting, and the transcription amount of the gene of the microorganism or the production amount of the protein by the microorganism is compared with that of a parent strain.

The fact that the microorganism produced by the above method is a microorganism having an enhanced activity of the protein according to any one of the above [1] to [6] and having improved productivity of an oligosaccharide having a Lewis X skeletons as compared with a parent strain can be confirmed by appropriately diluting a culture solution after culturing the microorganism, centrifuging the culture solution, and analyzing an oligosaccharide having a Lewis X skeleton and contained in a supernatant or in bacterial cells using a carbohydrate analyzer or a high performance liquid chromatograph mass spectrometer to be described later, thereby comparing the oligosaccharide having a Lewis X skeleton with that of the parent strain. In the present description, the "productivity of the oligosaccharide having a Lewis X skeleton" refers to an ability of the microorganism to accumulate the oligosaccharide having a Lewis X skeleton produced by the microorganism inside and/or outside the bacterial cells.

The above-described microorganism has an enhanced activity of the protein according to any one of the above [1] to [6] as compared with a parent strain, so that fucose can be selectively transferred to an N-acetylglucosamine site of a substrate, and the productivity of the oligosaccharide having a Lewis X skeleton can be improved. Examples of such a microorganism include a TROS/pPgsFucT1 or FUC/pPgsFucT1 strain having enhanced expression of the FucT1 gene, a FUC/pFucT-D strain having enhanced expression of the FucT-D gene, a FUC/pFucT-A strain having enhanced expression of the FucT-A gene, and a FUC/pFucT-E strain having enhanced expression of the FucT-E gene, as described below in Examples.

In a microorganism having an enhanced activity of a FucT1 protein, a FucT-D protein, a FucT-A protein, or a FucT-E protein, which is an example of such a microorganism, the activity of α1,3-fucose transferase capable of selectively transferring fucose to an N-acetylglucosamine site can be enhanced, and the productivity of the oligosaccharide having a Lewis X skeleton can be improved.

### 2. Method for Producing Oligosaccharide

Examples of a method for producing an oligosaccharide of the present invention (hereinafter, also referred to as a method of the present invention) include the following method.

A method for producing an oligosaccharide, including: preparing the microorganism described above in 1.; and producing an oligosaccharide in a culture using the microorganism.

In the method of the present invention, a desired oligosaccharide is preferably an oligosaccharide having a Lewis X skeleton, and more preferably an oligosaccharide having an LNFPIII skeleton or an oligosaccharide in which L-fucose is α1,3-linked to 3-position of at least one N-acetylglucosamine contained in a Para-LNnH skeleton.

In the method of the present invention, when the desired oligosaccharide is an oligosaccharide having an LNFPIII skeleton, the oligosaccharide is preferably at least one of LNFPIII and LNnDFHII.

In the method of the present invention, when the desired oligosaccharide is the oligosaccharide in which L-fucose is α1,3-linked to 3-position of at least one N-acetylglucosamine contained in a Para-LNnH skeleton, examples of such an oligosaccharide include oligosaccharides having a structure shown in FIG. 3.

The method for culturing the microorganism described in 1. can be performed according to a usual method used for culturing a microorganism.

As a culture medium for culturing a microorganism, any of a natural culture medium and a synthetic culture medium may be used as long as the culture medium contains a carbon source, a nitrogen source, an inorganic salt, and the like that can be assimilated by the microorganism and can efficiently culture the transformant.

Any carbon source may be used as long as it can be assimilated by the microorganism, and examples thereof include saccharides such as glucose, fructose, sucrose, molasses containing these, starch, or a starch hydrolysate, organic acids such as acetic acid or propionic acid, or alcohols such as glycerol, ethanol, or propanol.

Examples of the nitrogen source include ammonium salts of inorganic acids or organic acids such as ammonia, ammonium chloride, ammonium sulfate, ammonium acetate, or ammonium phosphate, other nitrogen-containing compounds thereof, peptone, a meat extract, a yeast extract, a corn steep liquor, a casein hydrolyzate, a soybean meal, a soybean meal hydrolyzate, various fermented bacterial cells, and digestive products thereof.

Examples of the inorganic salt include potassium primary phosphate, potassium secondary phosphate, magnesium phosphate, magnesium sulfate, sodium chloride, ferrous sulfate, manganese sulfate, copper sulfate, and calcium carbonate.

As the microorganism of the present invention used in the method for producing an oligosaccharide, a microorganism having an ability to produce glucose, lactose, lactose monohydrate, or the like may be used.

In the method for producing an oligosaccharide, glucose, lactose, lactose monohydrate, etc. may be added to the culture medium during culture.

When the microorganism of the present invention used in the method for producing an oligosaccharide does not have the ability to produce GDP-fucose and/or a receptor carbohydrate, which is a substrate for the oligosaccharide having a Lewis X skeleton, GDP-fucose and/or a receptor carbohydrate may be added to the culture medium.

In the method for producing an oligosaccharide, instead of adding glucose, lactose, lactose monohydrate, or a receptor carbohydrate to the culture medium during culture, glucose, lactose, lactose monohydrate, or a receptor carbohydrate may be supplied to the microorganism of the present invention by co-culturing a microorganism having an ability to produce glucose, lactose, lactose monohydrate, or a receptor carbohydrate from sugar simultaneously with the microorganism of the present invention.

In the method for producing an oligosaccharide, β-galactosidase and WcaJ are preferably absent in the culture medium.

The culture is generally preferably performed under aerobic conditions such as shaking culture, deep aeration stirring culture, or Jar fermentor culture. The culture temperature is generally 30°C to 37°C, and the culture time is generally 24 hours to 3 days. The pH of the culture solution during culture is generally maintained at 6.0 to 8.0. The pH is adjusted using an inorganic or organic acid, an alkaline solution, urea, calcium carbonate, ammonia, or the like.

An oligosaccharide can be produced by producing the oligosaccharide in a culture by the above-described culture.

Generally, the oligosaccharide can be collected from a supernatant after centrifugation of the culture. When the oligosaccharide is accumulated in bacterial cells, for examples, the oligosaccharide can be collected, by using an ion exchange resin method or the like, from a supernatant obtained by crushing the bacterial cells with ultrasonic waves or the like and removing the bacterial cells by centrifugation.

A target oligosaccharide can also be produced by adding another saccharide to the oligosaccharide in the culture or the collected oligosaccharide.

### [Analysis Example]

### (1) Analysis and Quantification of LNFPIII, 3FL, LNFPVI, or Lactose

In Examples, analysis and quantification of LNFPIII, 3FL, LNFPVI, or lactose were performed by the following procedure.

A culture solution containing microorganisms after culture was centrifuged, and a supernatant was collected. Precipitated bacterial cells were suspended in water in an amount equal to that of the original culture solution, further, the bacterial cells were disrupted by adding an equal amount of chloroform, followed by centrifugation, and a supernatant aqueous phase was used as a bacterial cell fraction. LNFPIII, 3FL, LNFPVI, or lactose contained in the supernatant and/or the bacterial cell fraction was analyzed by a carbohydrate analyzer ICS-5000 (manufactured by Thermo Fisher Scientific K.K.).

### [Analysis Conditions]

Column: CarboPAC PA1
Column temperature: 25°C
Mobile phase:
   (mobile phase A) water
   (mobile phase B) 500 mmol/L sodium hydroxide
   (mobile phase C) 300 mmol/L sodium acetate
Mobile phase A, mobile phase B, and mobile phase C mixing ratio:
   (0 minutes to 10 minutes) 80:20:0
   (10 minutes to 18 minutes) gradient from 80:20:0 to 70:20:10
   (18 minutes to 35 minutes) gradient from 70:20:10 to 0:20:80
   (35 minutes to 40 minutes) 0:20:80
   (40 minutes to 50 minutes) 80:20:0
   Flow rate: 1.0 mL/min
   Detector: pulsed amperometric detector

### (2) Analysis and Quantification of Lewis X or LNnDFHII

In Examples, analysis and quantification of Lewis X or LNnDFHII were performed by the following procedure.

A supernatant and/or a bacterial cell fraction was prepared from a culture solution containing cultured microorganisms in the same manner as in the above (1). Lewis X or LNnDFHII contained in the supernatant and/or the bacterial cell fraction was analyzed by UFLC&LCMS-8040 (manufactured by SHIMADZU).

### [Analysis Conditions]

Column: Coregel 87H3 (7.8 × 300 mm)
Column temperature: 40°C
Mobile phase: 0.1% formic acid water
Gradient condition: isocratic elution
Analysis time: 25 minutes
Flow rate: 0.4 ml/min
Injection amount: 10 µL
Detection: SIM mode

### [Example]

Examples of the invention are shown below, but the present invention is not limited to these Examples.

### [Example 1] Acquisition of α1,3-fucosyl transferase Useful for Producing LNFPIII

Using productivity of 3FL or Lewis X as an indicator, α1,3-fucosyl transferase showing high substrate specificity for N-acetyllactosamine was screened.

### (1) Construction of Host Strain for Evaluation

### <Acquisition of DNA Fragment to be Used as Marker for Gene Deletion>

PCR was performed using DNAs consisting of the nucleotide sequences represented by SEQ ID NOs: 33 and 34 as a primer set and pCatSac (Appl Environ Microbiol (2013) 79, 3033-3039) as a template to obtain a cat-sacB fragment containing a chloramphenicol-resistant cat gene and a sucrose sensitive sacB gene.

### <Construction of Escherichia Coli Lacking β-galactosidase Activity, Lactose Permease Activity, and Colanic Acid Synthesis Activity>

Escherichia coli deficient in DNA encoding β-galactosidase (hereinafter, referred to as lacZ gene), DNA encoding lactose permease (hereinafter, referred to as lacY gene), and DNA encoding a colanic acid production-related protein (hereinafter, referred to as wcaJ, wzxC, wcaK, wcaL, or wcaM gene) was constructed by the following method. Note that lacZ and lacY (hereinafter, referred to as lacZY), and wcaJ, wzxC, wcaK, wcaL, and wcaM (hereinafter, referred to as wcaJ-wzxC-wcaKLM) each form an operon on the Escherichia coli genome.

PCR was performed using, as a template, a genomic DNA of the Escherichia coli W3110 strain prepared by a common method and using, as a primer set, DNAs consisting of the nucleotide sequence represented by "Primer set" in Table 1 to amplify each DNA fragment.

**[Table 1]**

| Primer set (SEQ ID NO:) | Amplified DNA fragment | Remarks |
|---|---|---|
| 35 and 36 | lacZ upstream 1 | Sequences of nucleotide sequences represented by SEQ ID NOs: 33 and 35 at 5' ends are complementary |
| 37 and 38 | lacY downstream 1 | Sequences of nucleotide sequences represented by SEQ ID NOs: 34 and 37 at 5' ends are complementary |
| 36 and 39 | lacZ upstream 2 | Sequences of nucleotide sequences represented by SEQ ID NOs: 39 and 40 at 5' ends are complementary |
| 38 and 40 | lacY downstream 2 | |

The lacZ upstream 1 and lacZ upstream 2 contain a region from an initiation codon of the lacZ gene to about 1000 bp upstream of the initiation codon. The lacY downstream 1 and lacY downstream 2 contain a region from about 50 bp to about 1000 bp downstream of a stop codon of the lacY gene.

PCR was performed using, as a template, a mixture of the lacZ upstream 1, the lacY downstream 1, and the cat-sacB fragment at an equimolar ratio and using, as a primer set, DNAs consisting of the nucleotide sequences represented by SEQ ID NOs: 36 and 38 to obtain a DNA (hereinafter, referred to as lacZY::cat-sacB) fragment consisting of a sequence with the cat-sacB fragment inserted in a region around the lacZ and lacY genes.

PCR was performed using, as a template, a mixture of the lacZ upstream 2 and the lacY downstream 2 at an equimolar ratio and using, as a primer set, DNAs consisting of the nucleotide sequences represented by SEQ ID NOs: 36 and 38 to obtain a DNA (hereinafter, referred to as ΔlacZY) fragment consisting of a sequence with the lacZ upstream and the lacY downstream directly linked to each other without lacZY.

The lacZY::cat-sacB fragment was introduced into a W3110 strain carrying a plasmid pKD46 comprising a gene encoding λ recombinase [Datsenko, K. A., Warner, B. L. L., Proc. Natl. Acad.Sci, USA, Vol. 97, 6640-6645, (2000)] by an electroporation method to obtain a transformant (a transformant with the lacZY gene substituted with lacZY::cat-sacB) exhibiting chloramphenicol resistance and sucrose sensitivity.

The ΔlacZY fragment was introduced into the transformant by the electroporation method to obtain a transformant (a transformant with lacZY::cat-sacB substituted with ΔlacZY) exhibiting chloramphenicol sensitivity and sucrose resistance. Among them, a transformant (a transformant without pKD46) exhibiting ampicillin sensitivity was further obtained. The transformant was named W3110ΔlacZY.

Similarly, PCR was performed using, as a template, the genomic DNA of the W3110 strain and using, as a primer set, DNAs consisting of the nucleotide sequences represented by "Primer set" in Table 2 to obtain each amplified DNA fragment.

**[Table 2]**

| Primer set (SEQ ID NO:) | Amplified DNA fragment | Remarks |
|---|---|---|
| 41 and 42 | wcaJ upstream 1 | Sequences of nucleotide sequences represented by SEQ ID NOs: 33 and 41 at 5' ends are complementary |
| 43 and 44 | wcaM downstream 1 | Sequences of nucleotide sequences represented by SEQ ID NOs: 34 and 43 at 5' ends are complementary |
| 42 and 45 | wcaJ upstream 2 | Sequences of nucleotide sequences represented by SEQ ID NOs: 45 and 46 at 5' ends are complementary |
| 44 and 46 | wcaM downstream 2 | |

The wcaJ upstream 1 and the wcaJ upstream 2 contain a region from an initiation codon of the wcaJ gene to about 1000 bp upstream of the initiation codon. The wcaM downstream 1 and the wcaM downstream 2 contain a region from a stop codon of the wcaM gene to about 1000 bp downstream of the stop codon.

PCR was performed using, as a template, a mixture of the wcaJ upstream 1, the wcaM downstream 1, and the cat-sacB fragment at an equimolar ratio and using, as a primer set, DNAs consisting of the nucleotide sequences represented by SEQ ID NOs: 42 and 44 to obtain a DNA (hereinafter, referred to as wcaJ-wzxC-wcaKLM::cat-sacB) fragment consisting of a sequence with the cat-sacB fragment inserted in a region around the wcaJ-wzxC-wcaKLM operon.

PCR was performed using, as a template, a mixture of the wcaJ upstream 2 and the wcaM downstream 2 at an equimolar ratio and using, as a primer set, DNAs consisting of the nucleotide sequences represented by SEQ ID NOs: 42 and 44 to obtain a DNA (hereinafter, referred to as ΔwcaJ-wzxC-wcaKLM) fragment consisting of a sequence with the wcaJ upstream and the wcaM downstream directly linked to each other without wcaJ-wzxC-wcaKLM.

The wcaJ-wzxC-wcaKLM::cat-sacB fragment was introduced into the W3110 ΔlacZY strain constructed as described above by the electroporation method to obtain a transformant (a transformant with wcaJ-wzxC-wcaKLM substituted with wcaJ-wzxC-wcaKLM::cat-sacB) exhibiting chloramphenicol resistant and sucrose sensitivity.

The ΔwcaJ-wzxC-wcaKLM fragment was introduced into the transformant by the electroporation method to obtain a transformant (a transformant with wcaJ-wzxC-wcaKLM::cat-sacB substituted with ΔyhbJ) exhibiting chloramphenicol sensitivity and sucrose resistance. Further, a transformant (a transformant without pKD46) exhibiting ampicillin sensitivity was obtained. The transformant was named W3110ΔlacZYΔwcaJM strain.

### <Construction of Microorganism having Enhanced Expression of Transporter Gene>

Escherichia coli having a plasmid for expressing a gene encoding a transporter gene (hereinafter referred to as MdfA) belonging to the drug:H⁺ antiporter-1 family derived from the W3110 strain, consisting of the amino acid sequence represented by SEQ ID NO:28, was constructed by the following method.

PCR was performed using DNAs consisting of the nucleotide sequences represented by SEQ ID NOs: 47 and 48 as a primer set and the genomic DNA of the W3110 strain prepared by a common method as a template to obtain an mdfA fragment. PCR was performed using DNAs consisting of the nucleotide sequences represented by SEQ ID NOs: 49 and 50 as a primer set and using a plasmid pMW118 (manufactured by NIPPON GENE CO., LTD.) as a template to obtain a vector fragment of about 4.1 kb. The nucleotide sequences represented by SEQ ID NOs: 47 and 49 and SEQ ID NOs: 48 and 50 comprise complementary sequences at each 5' end.

The mdfA fragment and the vector fragment obtained above were ligated using an In-Fusion HD Cloning Kit (manufactured by Takara Bio Inc.) to obtain an MdfA expression plasmid pMW118_mdfA. The W3110ΔlacZYΔwcaJM strain constructed in Example 1 (1) was transformed with the expression plasmid pMW 118_mdfA to construct Escherichia coli having pMW 118_mdfA, which was named FUC strain.

### (2) Construction of Microorganism having α1,3-Fucosyl transferase Activity

Escherichia coli having a plasmid for expressing a gene encoding various α1,3-fucosyl transferases and placed at the downstream of the uspA promoter was constructed by the following method.

### <Construction of Expression Vector>

PCR was performed using, as a template, the genomic DNA of the W3110 strain prepared by a common method and using, as a primer set, DNAs consisting of the nucleotide sequences represented by "Primer set" in Table 3 to obtain each amplified DNA fragment.

**[Table 3]**

| Primer set (SEQ ID NO:) | Amplified DNA fragment | Remarks |
|---|---|---|
| 53 and 54 | rcsA | Sequences of nucleotide sequences represented by SEQ ID NOs: 54 and 55 at 5' ends are complementary |
| 55 and 56 | lacY | |

PCR was performed using, as a template, a mixture of the rcsA fragment and the lacY fragment at an equimolar ratio and using, as a primer set, DNAs consisting of the nucleotide sequences represented by SEQ ID NOs: 53 and 56 to obtain a DNA (hereinafter, referred to as rcsA-lacY) fragment with the two fragments ligate.

PCR was performed using, as a primer set, DNAs consisting of the nucleotide sequences represented by SEQ ID NOs: 51 and 52 and using, as a template, a plasmid pUAKQE 31 (Appl. Environ. Microbiol. 2007, 73: 6378-6385) to obtain a vector fragment of about 4.7 kb.

The nucleotide sequences represented by SEQ ID NOs: 51 and 53 and SEQ ID NOs: 52 and 56 comprise complementary sequences at each 5' end.

The rcsA-lacY fragment and the vector fragment obtained above were ligated using an In-Fusion HD Cloning Kit (manufactured by Takara Bio Inc.) to obtain an expression vector pUAKQE-rcsA-lacY.

### <Construction of Plasmid for Expressing α1,3-Fucosyl transferase>

PCR was performed using, as a primer set, DNAs consisting of the nucleotide sequences represented by "Primer set" in Table 4 and using, as a template, a DNA described in "Template" in Table 4 to obtain each amplified DNA fragment.

**[Table 4]**

| Primer set (SEQ ID NO:) | Template | Amplified DNA fragment |
|---|---|---|
| 58 and 59 | DNA represented by SEQ ID NO: 3 | HpFutA (SEQ ID NO: 3) |
| 60 and 61 | Genomic DNA of Bacteroides nordii JCM 12987 | BnFucT (SEQ ID NO: 5) |
| 62 and 63 | Genomic DNA of Bacteroides salyersiae JCM 12988 | BsFucT (SEQ ID NO: 7) |
| 64 and 65 | Genomic DNA of Parabacteroides goldsteinii JCM 13446 | PgsFucT1 (SEQ ID NO: 1) |
| 66 and 67 | Genomic DNA of Parabacteroides goldsteinii JCM 13446 | PgsFucT2 (SEQ ID NO: 9) |
| 68 and 69 | Genomic DNA of Bacteroides fragilis ATCC 25285 | BfFucT1 (SEQ ID NO: 11) |
| 70 and 71 | Genomic DNA of Bacteroides fragilis ATCC 25285 | BfFucT2 (SEQ ID NO: 13) |
| 72 and 73 | DNA represented by SEQ ID NO: 15 | MFucT (SEQ ID NO: 15) |

Various genomic DNAs were prepared by a common method. The DNA represented by SEQ ID NO: 3 was a nucleotide sequence of a gene encoding α1,3-fucosyl transferase, derived from the Helicobacter pylori 26695 strain and represented by SEQ ID NO: 4, and was prepared by artificial synthesis. The DNA represented by SEQ ID NO: 15 was a DNA in which a nucleotide sequence of the gene encoding α1,3-fucosyl transferase derived from the Mediterranea sp. An20 strain and represented by SEQ ID NO: 16 was codon-optimized for expression in Escherichia coli, and was prepared by artificial synthesis.

PCR was performed using the expression vector pUAKQE-rcsA-lacY constructed by the above-described method as a template and DNAs consisting of the nucleotide sequences represented by SEQ ID NOs: 51 and 57 as a primer set to obtain a vector fragment of about 6.7 kb.

The nucleotide sequences represented by SEQ ID NOs: 51, 58, 60, 62, 64, 66, 68, 70, and 72, and SEQ ID NOs: 57, 59, 61, 63, 65, 67, 69, 71, and 73 contain complementary sequences at each 5' end.

The various amplified DNA fragments and vector fragments obtained above were ligated using an In-Fusion HD Cloning Kit (manufactured by Takara Bio Inc.) to construct plasmids expressing various α1,3-fucosyl transferases, namely pHpFutA, pBnFucT, pBsFucT, pPgsFucT1, pPgsFucT2, pBfFucT1, pBfFucT2, and pMFucT.

### <Construction of Escherichia Coli having Plasmid for Expressing α1,3-Fucosyl transferase>

Using the above-obtained plasmid for expressing α1,3-fucosyl transferase, pHpFutA, pBnFucT, pBsFucT, pPgsFucT1, pPgsFucT2, pBfFucT1, pBfFucT2, and pMFucT, and pUAKQE-rcsA-lacY as a vector control, the FUC strain constructed in Example 1 (1) was transformed to produce Escherichia colis having various plasmids, which were named FUC/pHpFutA strain, FUC/pBnFucT strain, FUC/pBsFucT strain, FUC/pPgsFucT1 strain, FUC/pPgsFucT2 strain, FUC/pBfFucT1 strain, FUC/pBfFucT2 strain, FUC/pMFucT strain, and FUC/Ctrl strain, respectively.

### (3) Productivity Evaluation of 3FL or Lewis X

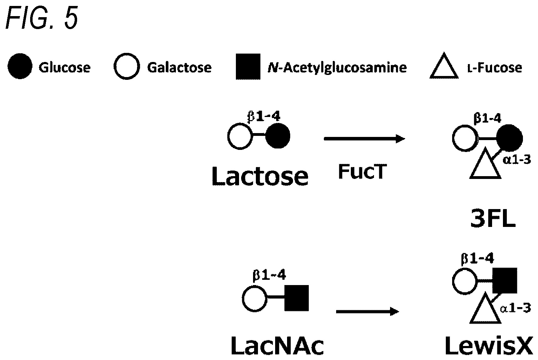

For the FUC/pHpFutA strain, the FUC/pBnFucT strain, the FUC/pBsFucT strain, the FUC/pPgsFucT1 strain, the FUC/pPgsFucT2 strain, the FUC/pBfFucT1 strain, the FUC/pBfFucT2 strain, the FUC/pMFucT strain, and the FUC/Ctrl strain, which were obtained in above (2), productivity of 3FL or Lewis X was evaluated. FIG. 5 shows biosynthesis pathways of 3FL and Lewis X.

Each strain was cultured on an LB plate containing 100 mg/L kanamycin and 100 mg/L ampicillin at 37°C for 17 hours, then inoculated into a 14 mL plastic tube containing 2 mL of LB culture medium containing 100 mg/L kanamycin and 100 mg/L ampicillin, and cultured with shaking at 30°C for 15 hours. Thereafter, 0.2 mL of each obtained culture solution was inoculated into a large-sized test tube containing 4 mL of a production culture medium [glucose 30 g/L, lactose monohydrate or N-acetyllactosamine 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (except for glucose, lactose monohydrate, N-acetyllactosamine, and magnesium sulfate heptahydrate adjusted to pH 7.2 by aqueous sodium hydroxide, and then autoclaved) (aqueous solutions containing glucose, lactose monohydrate, N-acetyllactosamine, and magnesium sulfate heptahydrate were separately prepared, autoclaved, cooled, and then mixed)] containing 100 mg/L kanamycin and 100 mg/L ampicillin, and cultured with shaking at 30°C for 29 hours. IPTG was added to a final concentration of 1 mM, 6.5 hours after the start of culture.

When evaluating the productivity of 3FL, a production culture medium containing lactose monohydrate was used, and when evaluating the productivity of Lewis X, a production culture medium containing 10 g/L of N-acetyllactosamine was used.

After completion of the culture, the culture solution was centrifuged and appropriately diluted, and 3FL or Lewis X contained in the supernatant was analyzed by a carbohydrate analyzer ICS-5000 or UFLC&LCMS-8040. The results are shown in Table 5.

**[Table 5]**

| Strain | 3FL [g/L] | Lewis X [g/L] |
|---|---|---|
| FUC/pHpFutA | 0.33 | 3.79 |
| FUC/pBnFucT | 1.08 | 1.24 |
| FUC/pBsFucT | 0.03 | 0.85 |
| FUC/pPgsFucT1 | N.D. | 2.82 |
| FUC/pPgsFucT2 | N.D. | N.D. |
| FUC/pBfFucT1 | 0.88 | 3.45 |
| FUC/pBfFucT2 | 0.08 | 2.18 |
| FUC/pMFucT | 0.08 | 1.47 |
| FUC/Ctrl | N.D. | N.D. |

Most of the strains were able to produce either 3FL or Lewis X or neither, but only the FUC/pPgsFucT1 strain was able to produce Lewis X significantly while not producing 3FL at all. From this, it was suggested that the α1,3-fucosyl transferase PgsFucT1 derived from Parabacteroides goldsteinii showed high substrate specificity for N-acetyllactosamine and could not utilize lactose as a substrate.

Based on the above results, PgsFucT1 was selected as the α1,3-fucosyl transferase for producing LNFPIII and was subjected to the following tests.

### [Example 2] Evaluation of Homologue Gene of PgsFucT 1

The homolog gene of PgsFucT1 selected in Example 1 was evaluated for usefulness in LNFPIII production.

### (1) Construction of Microorganism having Homologue Gene of PgsFucT 1

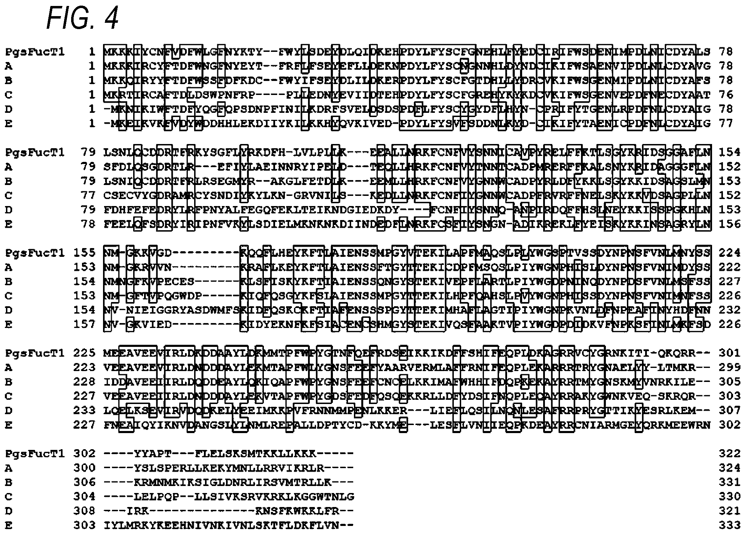

Escherichia coli having a homologue gene of PgsFucT 1 was constructed by the following method. As the homologue gene of PgsFucT1, five types of genes represented by SEQ ID NOs: 17 to 26 were used. FIG. 4 shows an alignment of the amino acid sequences encoded by each homologue gene.

PCR was performed using a DNA described in "Template" in Table 6 as a template and DNAs consisting of the nucleotide sequences represented by "Primer set" in Table 6 as a primer set to amplify each DNA fragment.

**[Table 6]**

| Primer set (SEQ ID NO:) | Template | Amplified DNA fragment |
|---|---|---|
| 74 and 75 | DNA represented by SEQ ID NO: 17 | FucT-A (SEQ ID NO: 17) |
| 76 and 77 | DNA represented by SEQ ID NO: 19 | FucT-B (SEQ ID NO: 19) |
| 78 and 79 | DNA represented by SEQ ID NO: 21 | FucT-C (SEQ ID NO: 21) |
| 80 and 81 | DNA represented by SEQ ID NO: 23 | FucT-D (SEQ ID NO: 23) |
| 82 and 83 | DNA represented by SEQ ID NO: 25 | FucT-E (SEQ ID NO: 25) |

The DNA represented by SEQ ID NO: 17 was a nucleotide sequence of a gene encoding α1,3-fucosyl transferase derived from the Parabacteroides sp. BX2 strain and represented by SEQ ID NO: 18, and was prepared by artificial synthesis. The DNA represented by SEQ ID NO: 19 was a nucleotide sequence of a gene encoding α1,3-fucosyl transferase derived from the Parabacteroides sp. HGS0025 strain and represented by SEQ ID NO: 20, and was prepared by artificial synthesis. The DNA represented by SEQ ID NO: 21 was a nucleotide sequence of a gene encoding α1,3-fucosyl transferase derived from the Parabacteroides bouchesdurhonensis strain Marseille-P3763 strain and represented by SEQ ID NO: 22, and was prepared by artificial synthesis. The DNA represented by SEQ ID NO: 23 was a nucleotide sequence of a gene encoding α1,3-fucosyl transferase derived from the Gramella sp. BOM4 strain and represented by SEQ ID NO: 24, and was prepared by artificial synthesis. The DNA represented by SEQ ID NO: 25 was a nucleotide sequence of a gene encoding α1,3-fucosyl transferase derived from the Lachnospiraceae bacterium NLAE-zl-G231 strain and represented by SEQ ID NO: 26, and was prepared by artificial synthesis.

In this case, the nucleotide sequences represented by SEQ ID NOs: 51, 74, 76, 78, 80, and 82, and SEQ ID NOs: 57, 75, 77, 79, 81, and 83 comprise complementary sequences at each 5' end.

The various amplified DNA fragments obtained above and the pUAKQE-rcsA-lacY vector fragment prepared in Example 1 were ligated using an In-Fusion HD Cloning Kit (manufactured by Takara Bio Inc.) to construct plasmids expressing various homologous genes, namely pFucT-A, pFucT-B, pFucT-C, pFucT-D, and pFucT-E.

The FUC strain constructed in Example 1 (1) was transformed with the five plasmids obtained above to construct Escherichia colis having various plasmids, which were named FUC/pFucT-A strain, FUC/pFucT-B strain, FUC/pFucT-C strain, FUC/pFucT-D strain, and FUC/pFucT-E strain, respectively.

### (2) Productivity Evaluation of 3FL or Lewis X

For the FUC/pFucT-A strain, the FUC/pFucT-B strain, the FUC/pFucT-C strain, the FUC/pFucT-D strain, and the FUC/pFucT-E strain obtained in above (1), the productivity of 3FL or Lewis X was evaluated. As a control, the FUC/pPgsFucT1 strain and the FUC/Ctrl strain constructed in Example 1 (2) were used.

The culture method and culture conditions were as described in Example 1 (3). After completion of the culture, the culture solution was centrifuged and appropriately diluted, and 3FL or Lewis X contained in the supernatant was analyzed by a carbohydrate analyzer ICS-5000 or UFLC&LCMS-8040. The results are shown in Table 7.

**[Table 7]**

| Strain name | 3FL [g/L] | Lewis X [g/L] |
|---|---|---|
| FUC/pFucT-A | N.D. | 0.10 |
| FUC/pFucT-B | N.D. | N.D. |
| FUC/pFucT-C | N.D. | N.D. |
| FUC/pFucT-D | N.D. | 0.35 |
| FUC/pFucT-E | N.D. | 0.10 |
| FUC/pPgsFucT1 | N.D. | 2.82 |
| FUC/Ctrl | N.D. | N.D. |

As a result, although the strains having each homologue gene had LewisX productivity lower than that of PgsFucT 1, none of the strains showed 3FL productivity, indicating that the proteins encoded by each homologue gene could not utilize lactose as a substrate, similar to PgsFucT1.

### [Example 3] Identification of Effective Mutation Point

A crystal structure of the known Helicobacter pylori-derived α1,3-fucosyl transferase has been disclosed, and amino acid residues associated with interaction with N-acetyllactosamine have been predicted (J. Biol. Chem. 2007, 282, 9973-9982). With reference to this, amino acid residues that interact with N-acetyllactosamine were identified by alignment for PgsFucT1 and BfFucT1, which showed high LewisX productivity in Example 1. The effectiveness in LNFPIII production was evaluated for the sequence in which the identified amino acid residues were mutually substituted with the corresponding amino acid residues of PgsFucT 1 and BfFucT 1.

### (1) Construction of Microorganisms having Mutant PgsFucT1 and Mutant BfFucT1

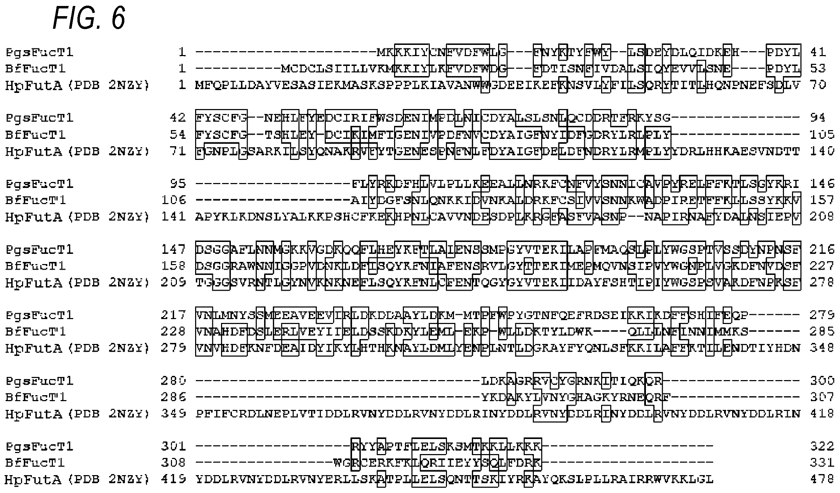

Escherichia colis having mutant PgsFucT1 and mutant BfFucT1 were constructed by the following method. FIG. 6 shows an alignment of the amino acid sequences encoding PgsFucT1, BfFucT and Helicobacter pylori-derived α1,3-fucosyl transferase.

PCR was performed using a DNA described in "Template" in Table 8 as a template and DNAs consisting of the nucleotide sequences represented by "Primer set" in Table 8 as a primer set to amplify each DNA fragment.

**[Table 8]**

| Primer set (SEQ ID NO:) | Template | Amplified DNA fragment | Remarks |
|---|---|---|---|
| 64 and 84 | pPgsFucT1 | PgsFucT1 N17D upstream | Sequences of nucleotide sequences represented by SEQ ID NOs: 84 and 85 at 5' ends are complementary |
| 65 and 85 | | PgsFucT1 N17D downstream | |
| 64 and 86 | | PgsFucT1 S93L upstream | Sequences of nucleotide sequences represented by SEQ ID NOs: 86 and 87 at 5' ends are complementary |
| 65 and 87 | | PgsFucT1 S93L downstream | |
| 68 and 88 | pBfFucT1 | BfFucT1 D28N upstream | Sequences of nucleotide sequences represented by SEQ ID NOs: 88 and 89 at 5' ends are complementary |
| 69 and 89 | | BfFucT1 D28N downstream | |
| 68 and 90 | | BfFucT1 L104S upstream | Sequences of nucleotide sequences represented by SEQ ID NOs: 90 and 91 at 5' ends are complementary |
| 69 and 91 | | BfFucT1 L104S downstream | |

PCR was performed using, as a template, a mixture of PgsFucT 1 N17D upstream and PgsFucT 1 N17D downstream fragments at an equimolar ratio, and using, as a primer set, DNAs consisting of the nucleotide sequences represented by SEQ ID NOs: 64 and 65 to obtain a PgsFucT1 N17D fragment in which asparagine in the amino acid sequence of PgsFucT 1 represented by SEQ ID NO: 2 at position 17 was substituted with aspartic acid.

Similarly, PCR was performed using, as a template, a mixture of PgsFucT1 S93L upstream and PgsFucT1 S93L downstream fragments at an equimolar ratio, and using, as a primer set, DNAs consisting of the nucleotide sequences represented by SEQ ID NOs: 64 and 65 to obtain a PgsFucT 1 S93L fragment in which serine in the amino acid sequence of PgsFucT1 represented by SEQ ID NO: 2 at position 93 was substituted with leucine.

Similarly, PCR was performed using, as a template, a mixture of BfFucT1 D28N upstream and BfFucT 1 D28N downstream fragments at an equimolar ratio, and using, as a primer set, DNAs consisting of the nucleotide sequences represented by SEQ ID NOs: 68 and 69 to obtain a BfFucT1 D28N fragment in which aspartic acid in the amino acid sequence of BfFucT 1 represented by SEQ ID NO: 12 at position 28 was substituted with asparagine.

Similarly, PCR was performed using, as a template, a mixture of BfFucT1 L104S upstream and BfFucT 1 L104S downstream fragments at an equimolar ratio, and using, as a primer set, DNAs consisting of the nucleotide sequences represented by SEQ ID NOs: 68 and 69 to obtain a BfFucT1 L 104S fragment in which leucine in the amino acid sequence of BfFucT1 represented by SEQ ID NO: 12 at position 104 was substituted with serine.

The fragments obtained above and the pUAKQE-rcsA-lacY vector fragment prepared in Example 1 were ligated using an In-Fusion HD Cloning Kit (manufactured by Takara Bio Inc.) to construct plasmids expressing the each of mutant fragments, namely pPgsFucT1_N17D, pPgsFucT1_S93L, pBfFucT1_D28N, and pBfFucT1_L104S.

The FUC strain constructed in Example 1 (3) was transformed with the four plasmids obtained above to construct Escherichia colis having various plasmids, which were named FUC/pPgsFucT1_N17D strain, FUC/pPgsFucT1_S93L strain, FUC/pBfFucT1_D28N strain, and FUC/pBfFucT1_L104S strain, respectively.

### (2) Productivity Evaluation of 3FL or Lewis X

For the FUC/pPgsFucT1_N17D strain, the FUC/pPgsFucT1_S93L strain, the FUC/pBfFucT1_D28N strain, and the FUC/pBfFucT1_L104S strain obtained in the above (1), productivity of 3FL or Lewis X was evaluated. As a control, the FUC/pPgsFucT 1 strain, the FUC/pBfFucT 1 strain, and the FUC/Ctrl strain constructed in Example 1 (2) were used.

The culture method and culture conditions were as described in Example 1 (3). After completion of the culture, the culture solution was centrifuged and appropriately diluted, and 3FL or Lewis X contained in the supernatant was analyzed by a carbohydrate analyzer ICS-5000 or UFLC&LCMS-8040. The results are shown in Table 9.

**[Table 9]**

| Strain name | 3FL [g/L] | Lewis X [g/L] | 3FL/Lewis X generation ratio [%] |
|---|---|---|---|
| FUC/Ctrl | N.D. | N.D. | - |
| FUC/pPgsFucT1 | N.D. | 1.68 | 0 |
| FUC/pPgsFucT1_N17D | 0.01 | 3.24 | 0.3 |
| FUC/pPgsFucT1_S93L | N.D. | N.D. | - |
| FUC/pBfFucT1 | 0.78 | 3.40 | 23 |
| FUC/pBfFucT1_D28N | 0.07 | 0.57 | 12 |
| FUC/pBfFucT1_L104S | N.D. | 0.35 | 0 |

In the FUC/pPgsFucT1_N17D strain, an amount of Lewis X produced was increased by about twofold compared with the FUC/pPgsFucT1 strain having the wild-type PgsFucT1, but a trace amount of 3FL was detected. On the other hand, in the FUC/pPgsFucT1_S93L strain, neither Lewis X nor 3FL was detected.

The FUC/pBfFucT1_D28N strain and the FUC/pBfFucT1_L104S strain showed a large decrease in amount of Lewis X produced as compared with the FUC/pBfFucT1 strain having the wild BfFucT1, and showed a decrease in amount of 3FL produced relative to Lewis X.

From the above results, it was suggested that the substrate specificity for lactose or N-acetyllactosamine could be modified by substituting an asparagine residue in the amino acid sequence of PgsFucT 1 at position 17 or an aspartic acid residue in the amino acid sequence of BfFucT 1 at position 28 or a leucine residue at position 104 with another amino acid.

### [Example 4] Construction of Microorganism Used for Producing LNFPIII

Escherichia coli having a plasmid for expressing a gene encoding β1,4-galactosyltransferase derived from Helicobacter pylori (hereinafter, referred to as HpgalT) and a gene encoding β1,3-N-acetylglucosamine transferase derived from Neisseria polysaccharea (hereinafter, referred to as NplgtA) placed at the downstream of the lac promoter were constructed by the following method.

PCR was performed using a DNA described in "Template" in Table 10 as a template and DNAs consisting of the nucleotide sequences represented by "Primer set" in Table 10 as a primer set to amplify each DNA fragment.

**[Table 10]**

| Primer set (SEQ ID NO:) | Template | Amplified DNA fragment |
|---|---|---|
| 94 and 95 | Genomic DNA of Helicobacter pylori NCTC 11637 | HpgalT (SEQ ID NO: 29) |
| 96 and 97 | DNA represented by SEQ ID NO: 31 | NplgtA (SEQ ID NO: 31) |

The genomic DNA of the Helicobacter pylori NCTC11637 strain was prepared by a common method. The DNA represented by SEQ ID NO: 31 was a DNA in which a nucleotide sequence of the gene encoding a β1,3-N-acetylglucosamine transferase derived from a Neisseria polysaccharea ATCC43768 strain, represented by SEQ ID NO: 32 was codon-optimized for expression in Escherichia coli, and was prepared by artificial synthesis. The nucleotide sequences represented by SEQ ID NOs: 95 and 96 comprise complementary sequences at each 5' end.

PCR was performed using, as a template, a mixture of an HpgalT fragment and an NplgtA fragment at an equimolar ratio and using, as a primer set, DNAs consisting of the nucleotide sequences represented by SEQ ID NOs: 94 and 97 to obtain a DNA (hereinafter, referred to as HpgalT-NplgtA) fragment with the HpgalT fragment and the NplgtA fragment ligate.

PCR was performed using DNAs consisting of the nucleotide sequences represented by SEQ ID NOs: 92 and 93 as a primer set and using a plasmid pSTV29 (manufactured by Takara Bio Inc.) as a template to obtain a vector fragment of about 2.9 kb. The nucleotide sequences represented by SEQ ID NOs: 92 and 94 and SEQ ID NOs: 93 and 97 comprise complementary sequences at each 5' end.

The HpgalT-NplgtA fragment and vector fragment obtained above were ligated using an In-Fusion HD Cloning Kit (manufactured by Takara Bio Inc.) to obtain an expression plasmid pSTV _HpgalT-NplgtA.

The W3110ΔlacZYΔwcaJM strain constructed in Example 1 (2) was transformed with the expression plasmid pSTV_HpgalT-NplgtA to construct Escherichia coli having pSTV_HpgalT-NplgtA, which was named TROS strain.

The above-described TROS strain was transformed with the pHpFutA, pPgsFucT1, pBfFucT1 and pUAKQE-rcsA-lacY plasmids constructed in Example 1 (2) to obtain TROS/pHpFutA, TROS/pPgsFucT1, TROS/pBfFucT1 and TROS/Ctrl strains, respectively.

### [Example 5] Production of LNFPIII

For the TROS/pHpFutA, TROS/pPgsFucT1, TROS/pBfFucT1, and TROS/Ctrl strains obtained in Example 4, productivity of LNFPIII and a by-product saccharide was evaluated.

Each strain was cultured on an LB plate containing 100 mg/L kanamycin and 25 mg/L chloramphenicol at 37°C for 18 hours, then inoculated into a 14 mL plastic tube containing 2 mL of LB culture medium containing 100 mg/L kanamycin and 25 mg/L chloramphenicol, and cultured with shaking at 30°C for 15 hours. Thereafter, 0.2 mL of each obtained culture solution was inoculated into a large-sized test tube containing 4 mL of a production culture medium [glucose 30 g/L, lactose monohydrate 10 g/L, magnesium sulfate heptahydrate 2 g/L, dipotassium hydrogen phosphate 16 g/L, potassium dihydrogen phosphate 14 g/L, ammonium sulfate 2 g/L, citric acid 1 g/L, casamino acid 5 g/L, thiamine hydrochloride 10 mg/L, ferrous sulfate heptahydrate 50 mg/L, manganese sulfate pentahydrate 10 mg/L (except for glucose, lactose monohydrate, and magnesium sulfate heptahydrate adjusted to pH 7.2 by aqueous sodium hydroxide and then autoclaved) (aqueous solutions containing glucose, lactose monohydrate, and magnesium sulfate heptahydrate were separately prepared, autoclaved, cooled, and then mixed)] containing 100 mg/L of kanamycin and 25 mg/L of chloramphenicol, and cultured with shaking at 30°C for 29 hours. IPTG was added to a final concentration of 1 mM, 5 hours after the start of culture.

After completion of the culture, the culture solution was centrifuged and appropriately diluted, and LNFPIII, 3FL, LNnDFHII, or LNFPVI contained in the supernatant or bacterial cells was analyzed by a carbohydrate analyzer ICS-5000 or UFLC&LCMS-8040. The results are shown in Table 11. Both LNnDFHII and LNFPVI represent a peak relative value (%).

**[Table 11]**

| Strain name | LNFPIII [mg/L] | | 3FL [mg/L] | | LNnDFHII [%] | | LNFPVI [%] | |
|---|---|---|---|---|---|---|---|---|
| | Culture solution | Inside bacterial cells | Culture solution | Inside bacterial cells | Culture solution | Inside bacterial cells | Culture solution | Inside bacterial cells |
| TROS/Ctrl | N.D. | N.D. | N.D. | N.D. | N.D. | N.D. | N.D. | N.D. |
| TROS/pHpFutA | N.D. | N.D. | 80 | 126 | N.D. | 95 | N.D. | 24 |
| TROS/pBfFucT1 | 14 | 39 | 366 | 355 | 17 | 82 | 27 | 100 |
| TROS/pPgsFucT1 | 45 | 1072 | N.D. | N.D. | N.D. | 18 | N.D. | N.D. |

As a result, it was found that the strain expressing PgsFucT1 accumulated a larger amount of LNFPIII in any of the culture solution and the bacterial cells as compared with the strain expressing HpFutA or BfFucT1, which is known α1,3 fucosyl transferase. It was shown that the use of PgsFucT1 can reduce by-products such as 3FL, LNnDFHII, and LNFPVI.

Although the present invention has been described in detail with reference to specific embodiments, it will be apparent to those skilled in the art that various changes and modifications can be made without departing from the spirit and scope of the present invention. The present application is based on a Japanese Patent Application No. 2022-019024 filed on February 9, 2022, the entire contents of which are incorporated herein by reference. All references cited herein are incorporated in their entirety.

### SEQUENCE LISTING FREE TEXT

SEQ ID NO: 1: nucleotide sequence of FucT1 derived from Parabacteroides goldsteinii JCM 13446
SEQ ID NO: 2: amino acid sequence of FucT1 derived from Parabacteroides goldsteinii JCM 13446
SEQ ID NO: 3: nucleotide sequence of FutA derived from Helicobacter pylori 26695
SEQ ID NO: 4: amino acid sequence of FutA derived from Helicobacter pylori 26695
SEQ ID NO: 5: nucleotide sequence of FucT derived from Bacteroides nordii JCM 12987
SEQ ID NO: 6: amino acid sequence of FucT derived from Bacteroides nordii JCM 12987
SEQ ID NO: 7: nucleotide sequence of FucT derived from Bacteroides salyersiae JCM 12988
SEQ ID NO: 8: amino acid sequence of FucT derived from Bacteroides salyersiae JCM 12988
SEQ ID NO: 9: nucleotide sequence of FucT2 derived from Parabacteroides goldsteinii JCM 13446
SEQ ID NO: 10: amino acid sequence of FucT2 derived from Parabacteroides goldsteinii JCM 13446
SEQ ID NO: 11: nucleotide sequence of FucT1 derived from Bacteroides fragilis ATCC 25285
SEQ ID NO: 12: amino acid sequence of FucT1 derived from Bacteroides fragilis ATCC 25285
SEQ ID NO: 13: nucleotide sequence of FucT2 derived from Bacteroides fragilis ATCC 25285
SEQ ID NO: 14: amino acid sequence of FucT2 derived from Bacteroides fragilis ATCC 25285
SEQ ID NO: 15: nucleotide sequence of FucT derived from Mediterranea sp. An20
SEQ ID NO: 16: amino acid sequence of FucT derived from Mediterranea sp. An20
SEQ ID NO: 17: nucleotide sequence of FucT-A derived from Parabacteroides sp. BX2
SEQ ID NO: 18: amino acid sequence of FucT-A derived from Parabacteroides sp. BX2
SEQ ID NO: 19: nucleotide sequence of FucT-B derived from Parabacteroides sp. HGS0025
SEQ ID NO: 20: amino acid sequence of FucT-B derived from Parabacteroides sp. HGS0025
SEQ ID NO: 21: nucleotide sequence of FucT-C derived from Parabacteroides bouchesdurhonensis strain Marseille-P3763
SEQ ID NO: 22: amino acid sequence of FucT-C derived from Parabacteroides bouchesdurhonensis strain Marseille-P3763
SEQ ID NO: 23: nucleotide sequence of FucT-D derived from Gramella sp. BOM4
SEQ ID NO: 24: amino acid sequence of FucT-D derived from Gramella sp. BOM4
SEQ ID NO: 25: nucleotide sequence of FucT-E derived from Lachnospiraceae bacterium NLAE-zl-G231
SEQ ID NO: 26: amino acid sequence of FucT-E derived from Lachnospiraceae bacterium NLAE-zl-G231
SEQ ID NO: 27: nucleotide sequence of MdfA derived from E. coli W3110
SEQ ID NO: 28: amino acid sequence of MdfA derived from E. coli W3110
SEQ ID NO: 29: nucleotide sequence of GalT derived from Helicobacter pylori NCTC 11637
SEQ ID NO: 30: amino acid sequence of GalT derived from Helicobacter pylori NCTC 11637
SEQ ID NO: 31: nucleotide sequence of LgtA derived from Neisseria polysaccharea ATCC 43768
SEQ ID NO: 32: amino acid sequence of LgtA derived from Neisseria polysaccharea ATCC 43768
SEQ ID NOs: 33 and 34: nucleotide sequences of catsacB fragment amplification primers
SEQ ID NOs: 35 and 36: nucleotide sequences of lacZ upstream 1 amplification primers
SEQ ID NOs: 37 and 38: nucleotide sequences of lacY downstream 1 amplification primers
SEQ ID NO: 39: nucleotide sequence of lacZ upstream 2 amplification primer
SEQ ID NO: 40: nucleotide sequence of lacY downstream 2 amplification primer
SEQ ID NOs: 41 and 42: nucleotide sequences of wcaJ upstream 1 amplification primers
SEQ ID NOs: 43 and 44: nucleotide sequences of wcaM downstream 1 amplification primers
SEQ ID NO: 45: nucleotide sequence of wcaJ upstream 2 amplification primer
SEQ ID NO: 46: nucleotide sequence of wcaM downstream 2 amplification primer
SEQ ID NOs: 47 and 48: nucleotide sequences of mdfA fragment amplification primers
SEQ ID NOs: 49 and 50: nucleotide sequences of pMW118 fragment amplification primers
SEQ ID NOs: 51 and 52: nucleotide sequences of pUAKQE fragment amplification primers
SEQ ID NOs: 53 and 54: nucleotide sequences of rcsA fragment amplification primers
SEQ ID NOs: 55 and 56: nucleotide sequences of lacY fragment amplification primers
SEQ ID NO: 57: nucleotide sequence of pUAKQE-rcsA-lacY fragment amplification primer
SEQ ID NOs: 58 and 59: nucleotide sequences of HpFutA fragment amplification primers
SEQ ID NOs: 60 and 61: nucleotide sequences of BnFucT fragment amplification primers
SEQ ID NOs: 62 and 63: nucleotide sequences of BsFucT fragment amplification primers
SEQ ID NOs: 64 and 65: nucleotide sequences of PgsFucT1 fragment amplification primers
SEQ ID NOs: 66 and 67: nucleotide sequences of PgsFucT2 fragment amplification primers
SEQ ID NOs: 68 and 69: nucleotide sequences of BfFucT 1 amplification primers
SEQ ID NOs: 70 and 71: nucleotide sequences of BfFucT2 fragment amplification primers
SEQ ID NOs: 72 and 73: nucleotide sequences of MFucT fragment amplification primers
SEQ ID NOs: 74 and 75: nucleotide sequences of FucT-A fragment amplification primers
SEQ ID NOs: 76 and 77: nucleotide sequences of FucT-B fragment amplification primers
SEQ ID NOs: 78 and 79: nucleotide sequences of FucT-C fragment amplification primers
SEQ ID NOs: 80 and 81: nucleotide sequences of FucT-D fragment amplification primers
SEQ ID NOs: 82 and 83: nucleotide sequences of FucT-E fragment amplification primers
SEQ ID NO: 84: nucleotide sequence of PgsFucT1 N17D upstream amplification primer
SEQ ID NO: 85: nucleotide sequence of PgsFucT1 N17D downstream amplification primer
SEQ ID NO: 86: nucleotide sequence of PgsFucT 1 S93L upstream amplification primer
SEQ ID NO: 87: nucleotide sequence of PgsFucT 1 S93L downstream amplification primer
SEQ ID NO: 88: nucleotide sequence of BfFucT D28N upstream amplification primer
SEQ ID NO: 89: nucleotide sequence of BfFucT D28N downstream amplification primer
SEQ ID NO: 90: nucleotide sequence of BfFucT L104S upstream amplification primer
SEQ ID NO: 91: nucleotide sequence of BfFucT L104S downstream amplification primer
SEQ ID NOs: 92 and 93: nucleotide sequences of pSTV29 amplification primers
SEQ ID NOs: 94 and 95: nucleotide sequences of HpgalT fragment amplification primers
SEQ ID NOs: 96 and 97: nucleotide sequences of NplgtA fragment amplification primers

## Claims

1. A microorganism having an enhanced activity of a protein according to any one of the following [1] to [6] and improved productivity of an oligosaccharide having a Lewis X skeleton as compared with a parent strain:
[1] a protein consisting of the amino acid sequence represented by SEQ ID NO: 2,
[2] a mutant protein having an α1,3-fucosyl transferase activity and consisting of an amino acid sequence in which 1 to 20 amino acids are deleted, substituted, inserted, or added in the amino acid sequence represented by SEQ ID NO: 2,
[3] a homologous protein having an α1,3-fucosyl transferase activity and consisting of an amino acid sequence having an identity of 90% or more with the amino acid sequence represented by SEQ ID NO: 2,
[4] a protein consisting of the amino acid sequence represented by SEQ ID NO: 24,
[5] a protein consisting of the amino acid sequence represented by SEQ ID NO: 18, and
[6] a protein consisting of the amino acid sequence represented by SEQ ID NO: 26.

2. The microorganism according to claim 1, wherein the oligosaccharide having a Lewis X skeleton is an oligosaccharide having a lacto-N-fucopentaose III (LNFPIII) skeleton.

3. The microorganism according to claim 2, wherein the oligosaccharide having a LNFPIII skeleton is at least one of LNFPIII and lacto-N-neodifucohexaose II (LNnDFHII).

4. The microorganism according to claim 1, wherein the oligosaccharide having a Lewis X skeleton is an oligosaccharide in which L-fucose is α1,3-linked to 3-position of at least one N-acetyl glucosamine contained in a paralacto-N-neohexaose (Para-LNnH) skeleton.

5. A method for producing an oligosaccharide, the method comprising:
preparing the microorganism according to any one of claims 1 to 4; and producing an oligosaccharide in a culture using the microorganism.
